# EUROPEAN PATENT SPECIFICATION

(11) **EP 2 566 850 B1**
(45) Date of publication and mention of the grant of the patent: **11.04.2018**
(21) Application number: 11724733.8
(22) Date of filing: 06.05.2011
(51) Int. Cl.: C07D 223/16

(54) **INDUSTRIAL PROCESS FOR THE SYNTHESIS OF IVABRADINE HYDROBROMIDE SALT**
INDUSTRIEVERFAHREN FÜR DIE SYNTHESE VON IVABRADINHYDROBROMIDSALZ
PROCÉDÉ INDUSTRIEL POUR LA SYNTHÈSE DE SEL D'HYDROBROMIDE D'IVABRADINE

(30) Priority: 07.05.2010 HU 1000245
(43) Date of publication of application: 13.03.2013
(73) Proprietor: Richter Gedeon Nyrt., 1103 Budapest (HU)
(72) Inventor: UJVÁRI, Viktor, H-2230 Gyömrö (HU); BÓDI, József, H-1202 Budapest (HU); FARAGÓ, János, H-2730 Albertirsa (HU); SZÖKE, Katalin, H-1105 Budapest (HU); FAIGL, Ferenc, H-1114 Budapest (HU); NÉMET, Zoltán, H-8130 Enying (HU); TEMESVÁRI, Krisztina, H-1157 Budapest (HU); KISS, Róbert, H-2730 Albertirsa (HU); MÁTRAVÖLGYI, Béla, H-1183 Budapest (HU); KASSAI, Ferencné, H-1188 Budapest (HU); KISS-BARTOS, Dorottya, H-2730 Albertirsa (HU)
(74) Representative: Hoffmann Eitle
(86) International application number: PCT/HU2011/000044
(87) International publication number: WO 2011/138625

(56) References cited:
- EP-A1- 0 534 859
- EP-A1- 2 145 871
- WO-A1-2005/110993
- WO-A1-2009/124940
- WO-A1-2010/023383
- WO-A2-2008/065681
- WO-A2-2008/146308
- WO-A2-2009/153461
- DATABASE CA [Online] CHEMICAL ABSTRACTS SERVICE, COLUMBUS, OHIO, US; LIU, XIN ET AL: "Process for preparation of N-methyl-4,5- dimethoxybenzocyclobutenecarboxamide as Procoralan intermediate", XP000002657374, retrieved from STN Database accession no. 2010:364384 -& CN 101 671 265 A (SHANGHAI INSTITUTE OF MATERIA MEDICA, CHINESE ACADEMY OF SCIENCES, PEO) 17 March 2010 (2010-03-17)
- BUNNETT, J. F. ET AL: "Homocyclic ring closures via benzyne intermediates. A new synthesis of 1-substituted benzocyclobutenes", JOURNAL OF ORGANIC CHEMISTRY, vol. 27, 1962, pages 3836-3843, XP000002657257,
- KAMETANI, T. ET AL: "Syntheses of heterocyclic compounds. CDXCIV. Total synthesis of (+-)-xylopinine by thermolysis", TETRAHEDRON, vol. 29, no. 1, 1973, pages 73-76, XP000002657462,

## Description

### Field of the invention

The present invention relates to the synthesis of Ivabradine hydrobromide of formula (I) 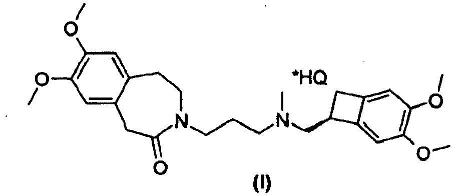 (wherein HQ = HBr),
the chemical name of which is: 3-{3-[((S)-3,4-dimethoxy-bicyclo[4.2.0]octa-1,3,5-triene-7-ylmethyl)-methyl-amino]-propyl}-7,8-dimethoxy-1,3,4,5-tetrahydro-2H-3-benzazepine-2-one hydrobromide, starting from 3-(2-bromo-4,5- dimethoxy-phenyl)-propionitrile of formula (**II**) and the new intermediates thereof.

The invention also relates to a certain crystalline form of ivabradine hydrobromide.

### Background of the invention

Ivabradine of formula (**I**) is an alternative drug for the treatment of stable angina pectoris in case of intolerance or contraindication for beta receptor blockers.

The first synthesis of Ivabradine of formula (**I**) is described in EP 534859 Patent of Servier.

According to this process the iodopropyl-benzazepinone derivative intermediate of formula (**XI**) is reacted with ((*S*)-3,4-dimethoxy-bicyclo[4.2.0]octa-1,3,5-triene-7-yl-methyl)-methyl-amine of formula (**VIIIb**) and the obtained product of formula (**Xb**) furthermore dehydro-Ivabradine is hydrogenated.

According to the known methods, described e.g. in: US4490369 the reactive iodopropyl-benzazepinone derivative of formula (**XI**) is synthesized starting from 1-(7,8-dimethoxy-1,3-dihydro-2H-3-benzazepine-2-on-3-yl)-3-chloro-propane reacting with sodium iodide and isolated in crystalline form.

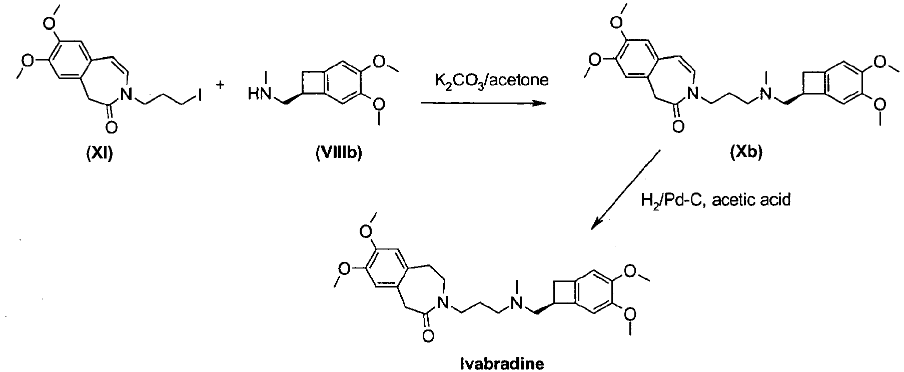

US 7176197 B2 Patent of Servier describes the synthesis of Ivabradine of formula (**I**) and the key intermediate is amino hydrochloride of formula (**VIII**).

According to this process Ivabradine of formula (**I**) is prepared by reductive alkylation in the presence of hydrogen and Pd/C catalyst starting form the compound of formula (**VIII**) with a protected aldehyde e.g.: 3-(2-[1,3]-dioxolan-2-yl-ethyl)-7,8-dimethoxy-1,3-dihydro-2H-3-benzazepine-2-one in one step. Paralelly, the dihydro-benzazepine moiety is saturated to tetrahydro-benzazepine structure in during the process.

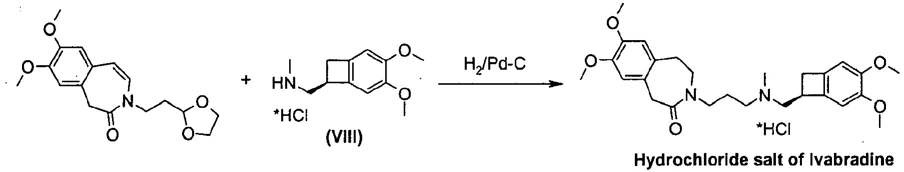

WO 2008 65681 PCT of Cadila describes an alternative synthetic method of Ivabradine of formula (**I**).

The key intermediate of this process is the methylamino-methyl-cyclobutane derivative of formula (**VIIIb**) which is reacted with 1-bromo-3-chloro-propane to the tertiary amine of formula (**XII**). Then the potassium salt of lactam of formula (**XIII**) iss alkylated with the obtained chloro-propyl-methyl-amine derivative.

According e.g.: to US4490369 Patent azetidine ring can also be produced under the conditions of alkylation of the chloropropyl tertiary amine of formula (**XII**).

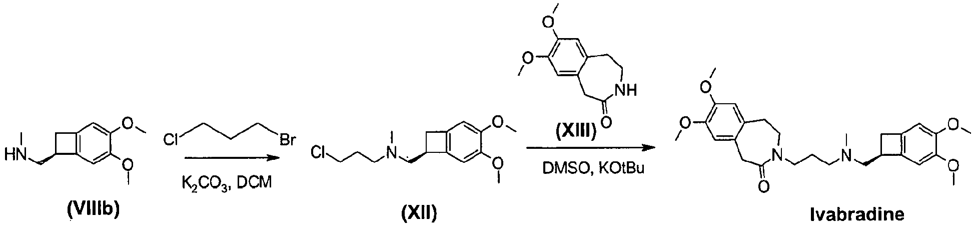

The key intermediate of the above processes is the enantiomer pure ((*S*)-3,4-dimethoxy-bicyclo[4.2.0]octa-1,3,5-triene-7-yl-methyl)-methyl-amine of formula (**VIIIb**).

EP 534859 Patent describes the preparation of this compound in enantiomer pure form. According to this process the compound of formula (**VIIIb**) is prepared from the racemic form by resolution with d-camphorsulfonic acid.

The camphorsulfonic salt is purified by repeated recrystallizations until the ee purity of 99% was obtained.

According to their examples the ratio of S/R enantiomers is only 84/16 and the yield is only 40%.

WO 2005 123659 PCT Patent Application of Servier describes a novel process for the preparation of enantiomer pure methylamino-methyl-benzocyclobutane derivative of formula (**VIII**).

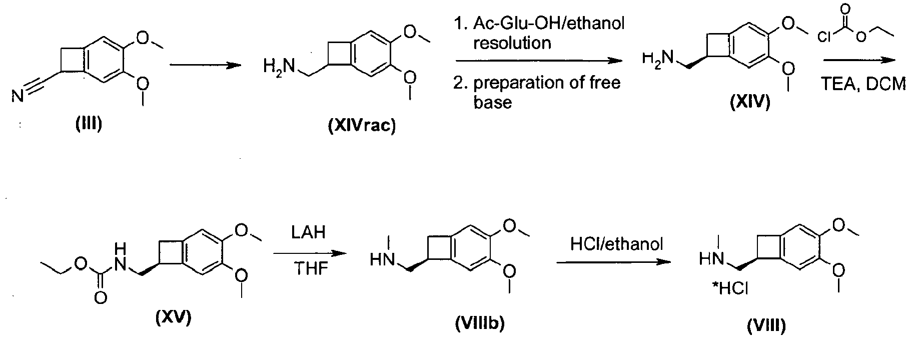

The racemic aminomethyl-benzocyclobutane derivative of formula (**XIVrac**) is prepared by reduction of the nitrile compound of formula (**III**)**.**

The diastereomeric salt is obtained by resolution of the racemic amine by N-acetyl-L-glutamic acid then the salt is purified by recrystallization.

The intermediate of formula (**XV**) is prepared from the enantiomeric pure compound of formula (**XIV**) and the formed urethane of formula (**XV**) is reduced by lithium-aluminumhydride (LAH).

The yield of the resolution is increased to ca.: 35-40%, the used resolving agent is cheaper than d-camphoric acid, however the 60 % of the racemic amine (**XIVrac**) is lost during the resolution, since the (*R*)-aminomethyl-benzocyclobutane is not regenerated.

The key intermediate of Ivabradine: ((*S*)-3,4-dimethoxy-bicyclo[4.2.0]octa-1,3,5-triene-7-yl-methyl)-methyl-amine compound of formula (**VIIIb**) and the salts thereof are prepared according to the above processes, mainly starting from the carbonitrile compound of formula (**III**). The synthesis of the compound of formula (**III**) can be carried out on different ways.

According to e.g.: US 4618683 Patent the starting material is 3-(2-bromo-4,5-dimethoxyphenyl)-propionitrile. Flammable and moisture sensitive alkali metal amide (e.g.: sodium or potassium) is used for the reaction in liquid ammonia at low temperature (generally at reflux temperature of ammonia: -33 °C).

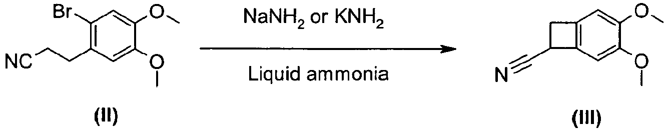

In the publication of Tetrahedron Letters, Vol.23, No.36, 3669-3672, 1982 study the flash vacuum pyrolysis (F.V.P.) of 2-methyl-benzyl-chlorides is described. In the examples the preparation of the nitrile compound of formula (**III**) is also described. The 2-methyl-4,5-dimethoxybenzaldehyde compound of formula (**XVI**) is reacted with the corrosive phosphorus pentachloride to yield the benzylidene-dichloride compound of formula (**XVII**). The dimethoxy-benzocyclobutane compound of formula (**XVIII**) was obtained by pyrolysis of compound of formula (**XVII**) at low pressure (0.1 mbar) and at high temperature with 60 % yield. The key intermediate of formula (**III**) is obtained by substitution the chloro atom with ciano group using tetraalkyl-ammonium-cianide (R₄NCN). The yield is 38 % yield.

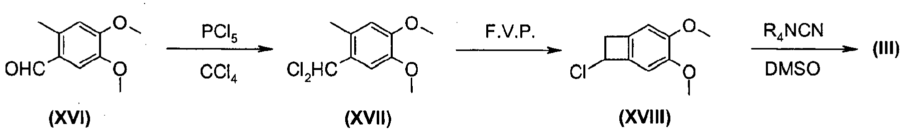

US 2010 16580A1 Patent Application of Servier describes the preparation of benzocyclobutane derivatives. In their process the bromophenyl-malonic ester-derivative of formula (**XIX**) is cyclized to diester of formula (**XX**) by palladium catalysis with 69% yield.

The diester of formula (**XX**) is purified by chromatography and then treated with trifluoro acetic acid followed by decarboxylation to yield dimethoxy-benzocyclobutenoic ester of formula (**XXI**).

This ester is reacted with aqueous solution of methylamine to form the amide of formula (**VIIrac**). This amide of formula (**VIIrac**) is reduced with borane/THF complex and the formed secondary amine of formula (**VIIIrac**) is isolated as the hydrochloride salt thereof.

The obtained secondary amine is resolved with camphorsulfonic acid and enantiomer pure amine of formula (**VIIIb**) was formed.

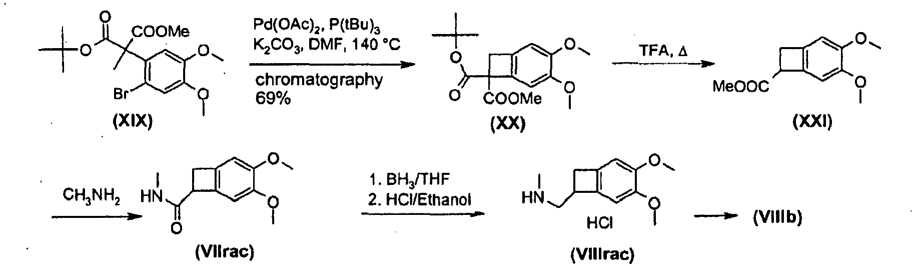

Only a few inorganic or organic salts of Ivabradine (**I**) are known. The hydrochloride salt is described in Servier's EP 534859 Patent.

Servier published more Patents which claim polymorphs and hydrate forms of Ivabradine (WO 2005/110993, WO 2006/092491, WO 2006/092492, WO 2006/092493, WO 2006/092494, WO 2007/042656, WO 2007/042657).

According to these, Ivabradine HCl exists in the form of at least one stable anhydrate and three different hydrates and the same metastable dehydrated hydrate forms.

Further known Ivabradine salts are the hydrobromide (WO 2009/124940) and oxalate (WO 2008/146308).
CN 1016711265 describes the chiral resolution of 4,5-dimethlxybenzeocyclobutene carboxylic acid with (R)-alpha-methylbenzyl amine (i.e., R-(+)-1-phenylethlyamine), in the preparation of ivabradine hydrochloride.
WO 2010/023383 present invention relates to a process for the optical resolution of (3,4-dimethoxy-bicyclo[4.2.0]octa-1,3,5-trien-7-yl)nitrile of formula (I) by chiral chromatography and to the application thereof in the synthesis of ivabradine, of its addition salts with a pharmaceutically acceptable acid and of their hydrates.
WO 2009/153461 is directed to a process for the synthesis of 7,8-dimethoxy-1,3-dihydro-2H-3-benzazepin-2-one, and application in the synthesis of ivabradine and addition salts thereof with a pharmaceutically acceptable acid.

### Brief description of the Invention

The present invention relates to a process for the synthesis of Ivabradine hydrobromide of formula (**I**) with HQ = HBr, the chemical name of which is: 3-{3-[((*S*)-3,4-dimethoxy-bicyclo[4.2.0]octa-1,3,5-triene-7-ylmethyl)-methyl-amino]-propyI}-7,8-dimethoxy-1,3,4,5-tetrahydro-2H-3-benzazepine-2-one hydrobromide, characterized by that
3-(2-bromo-4,5-dimethoxy-phenyl)-propionitrile of formula (**II**) is cyclized with alkali-alkylamide of formula M-NR⁴R⁵ (R⁴ and R⁵ are hydrogen, C₁-C₄ straight or branched alkyl group, substituted alkyl group respectively or R⁴ and R⁵ are C₄-C₆ alkylidene group which can be unsaturated heterocycle ring. R⁴ and R⁵ together can also be a C₄-C₆ alkylidene group which can form a heterocycle ring with nitrogen and M is an alkali metal), and
the obtained 3,4-dimethoxy-bicyclo[4.2.0]octa-1,3,5-triene-7-carbonitrile compound of formula (**III**) is hydrolyzed,
the obtained 3,4-dimethoxy-bicyclo [4.2.0]octa-1,3,5-triene-7-carboxylic acid compound of formula (**IV**) is resolved with chiral base of R¹R²R³N^{*} wherein R¹, R² and R³ are hydrogen, chiral or achiral C₁-C₄ alkyl, substituted chiral or achiral alkyl, chiral or achiral arylalkyl respectively. R¹R²R³N chiral base can also be natural alkaloid or the mixture, the derivatives or the mixture of derivatives thereof,
in case the diastereomer salt (**Vb**) crystallizes, then the carboxylic acid of formula (**VIb**) is released from the obtained crystalline diastereomer salt of formula (**Vb**)
or in case the diastereomer salt of formula (**Va**) crystallizes, then the carboxylic acid enantiomer of formula (**VIb**) is obtained from the filtrate,
the corresponding acid chloride of (*S*)-3,4-dimethoxy-bicyclo[4.2.0]octa-1,3,5-triene-7-carboxylic acid compound of formula (**VIb**) is formed *in situ* and without isolation it is reacted with methylamine,
the obtained (*S*)-3,4-dimethoxy-bicyclo[4.2.0]octa-1,3,5-triene-7-carboxylic acid - methylamide of formula (**VII**) is reduced
and ((*S*)-3,4-dimethoxy-bicyclo[4.2.0]octa-1,3,5-triene-7-yl-methyl)-methyl-amine hydrochloride of formula (**VIII**) is isolated, and it is reacted with 1-(7,8-dimethoxy-1,3-dihydro-2H-3-benzazepine-2-on-3-yl)-3-chloro-propane of formula (**IX**)
and the obtained 3-{3-[((S)-3,4-dimethoxy-bicyclo [4.2.0] octa-1,3,5-triene-7-ylmethyl)-methyl-amino]-propyl}-7,8-dimethoxy-1,3-dihydro-2H-3-benzazepine-2-on of formula (**X**) is isolated in the form of an acid addition salt of general formula HQ with HQ = NHO₃ or (COOH)₂ and purified by recrystallization.
and from 3-{3-[((S)-3,4-dimethoxy-bicyclo[4.2.0] octa-1,3,5-triene-7-ylmethyl)-methylamino]-propyl}-7,8-dimethoxy-1,3-dihydro-2H-3-benzazepine-2-one salt of formula (**X**) the base is released with K₂CO₃ with or without isolation,
the base is subjected to catalytic hydrogenation with Pd-C catalyst in an ethanol or methanol solvent at the pressure of 1-15 bar at temperature of 25-100°C, then the Ivabradine hydrobromide salt of formula (I) is isolated, wherein the crystallization Ivabradine hydrobromide is carried out by cooling the methanol or ethanol solution.

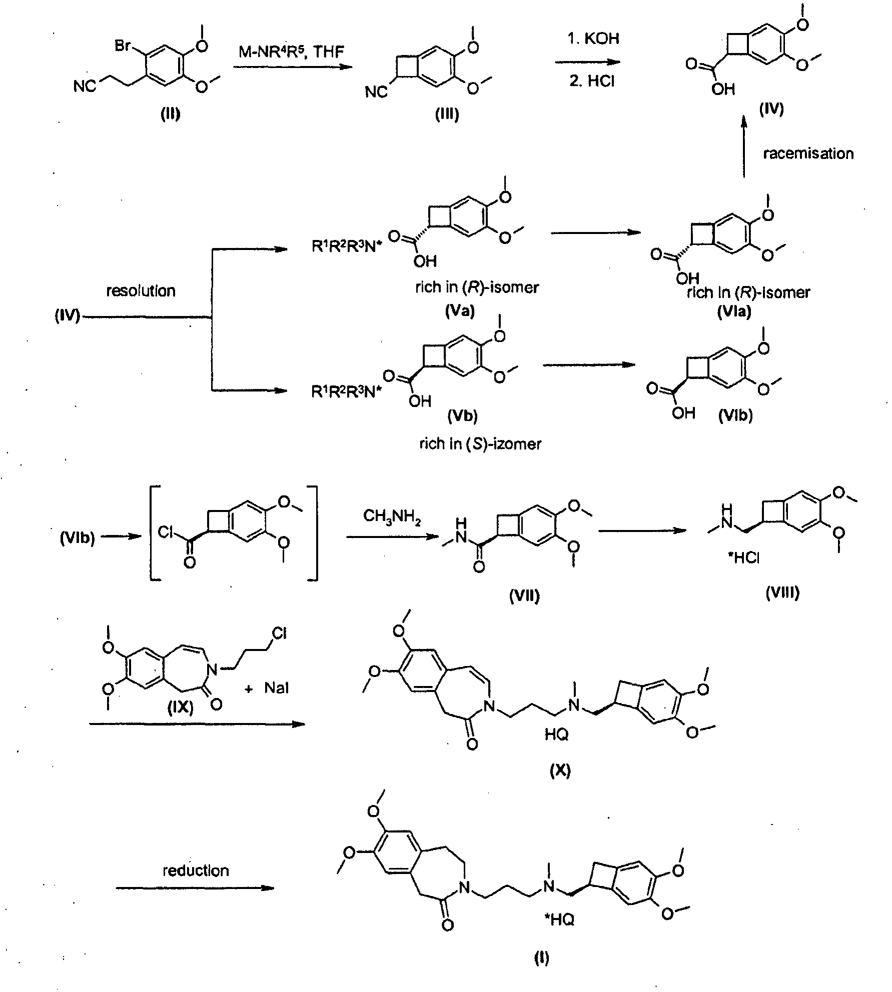

In another aspect, the present invention also relates to crystalline Ivabradine hydrobromide salt of formula (I) (HQ=HBr) characterized by at least one of the following characteristics: XRPD reflections measured at about: 5.0; 17.8; 18.5; 22.6; 23.8 and 25.0 °2θ IR absorption bands measured at about: 2930; 1664; 1522; 1463; 1219; and 1105 cm⁻¹ Raman absorption bands measured at about: 2948; 2909; 1607; 1588; 1317 and 701 cm⁻¹.

### Detailed description of the invention

The aim of the invention is to eliminate the disadvantages of the known synthetic processes.

According to this a new synthesis of Ivabradine salts of formula (**I**) is elaborated in high purity using new intermediates and chemical methods which can be scaled up to obtain new Ivabradine salts of formula (**I**).

For the cyclobutane ring formation novel ring closure reaction is used. The extreme reaction conditions and the use of hazardous chemicals are avoided in this way.

During our experiments it was found that cyclobutane-carbonitrile of formula (**III**) can be prepared by the reaction of 3-(2-bromo-4,5-dimethoxyphenyl)-propionitrile of formula (**II**) with metal-organic reagents (preferably with primer or secunder alkali salt thereof) in good yield.

Furthermore it was found that reacting the known racemic 3,4-dimethoxy-bicyclo[4.2.0]octa-1,3,5-triene-7-carboxylic acid of formula (**IV**) (e.g.: US4618683) with several chiral base, the (*R*)- or (*S*)-3,4-dimethoxy-bicyclo[4.2.0]octa-1,3,5-triene-7-carboxylic acid salt can be obtained in >80% de purity.

The (*S*)-3,4-dimethoxy-bicyclo[4.2.0]octa-1,3,5-triene-7-carboxylic acid salt can be purified by simple crystallization to obtain the product in high chemical and optical purity.

(*S*)-3,4-dimethoxy-bicyclo[4.2.0]octa-1,3,5-triene-7-carboxylic acid of formula (**VIb**) can be prepared from it's salt by deliberation of the acid.

In case the (*S*)-carboxylic acid enantiomer of formula (**VIb**) is enriched in the filtrate of salt formation, the enantiomer of formula (**VIb**) can be obtained by recrystallization of the mixture of the isomers of formula (**VIb>VIa**) or by selective precipitation from the aqueous solution of the alkali salt of the mixture.

The mixture of compounds of formula (**VIa-VIb**) is treated with base and then this racemized acid of formula (**IV**) was resolved again.

Furthermore the (*S*)-3,4-dimethoxy-bicyclo[4.2.0]octa-1,3,5-triene-7-carboxylic acid-N-methylamide of formula (**VII**) which is obtained from the carboxylic acid of formula (**VIb**) can be purified by simple recrystallization and in this way the methylamide of formula (**VII**) can be prepared with >99.8% ee enantiomeric purity.

The pure methyl-aminomethyl-hydrochloride of formula (**VIII**) can be prepared by reduction of the pure enantiomer of amide of formula (**VII**) in high yield and without racemisation.

In this way the Ivabradine of formula (**I**) and the salts thereof can be prepared in industrial using economical process and fewer synthetic steps.

The synthesis of new polymorphic forms and solvates of a pharmaceutically active compound provides a new opportunity to improve the physical properties of a pharmaceutical product. It enlarges the repertoire of materials that a formulation scientist has available for designing, for example, a pharmaceutical dosage form of a drug with a targeted release profile or other desired characteristic.

A different salt and / or polymorph may be preferred for large scale production e.g. due to its greater solubility and/or better flowing and filtration properties, and on the other hand it may have superior pharmacokinetics.

At room temperature Ivabradine is a semi-transparent, honey-like, viscous material. The purification and the technological use thereof are extremely difficult.

It is known for a qualified person that salt formation can overcome such problems.

The new structures obtained by salt formation can have new energetic relations which result in higher melting point and greater stability, and thus the material becomes more suitable for pharmaceutical use.

As it was mentioned above just 3 acid addition salts of Ivabradine (I) base are described in the prior art (hydrogen chloride; hydrogen bromide and oxalate).

Forming new salts of Ivabradine opens up new possibilities for the pharmaceutical usage of this active ingredient. New salts may have better dissolution properties, specific advantage of which can emerge only at the technological utilization thereof. New salts may show greater stability and / or better compatibility with the excipients of specific formulations; these properties, however, cannot be anticipated. The knowledge of the physico-chemical properties of new salts enlarges the repertoire the formulation scientist has to develop new and innovative drug products.

New solid forms (polymorphs, hydrates and other solvates, as well as amorphous form) of various salts imply similar practical advantages. In some cases different solid forms of the same compound differ from each other so much in respect of their technological properties, manufacturing and utilization, as it were solid forms of different compounds. Therefore it cannot be predicted which solid form will possess specific advantages over the others.

According to the fact that Ivabradine is a relatively strong base it could have been thought that it forms salts easily with organic and inorganic acids.

Our thorough research revealed, however, that the majority of the acids form amorphous salt product with the free base, and it was not possible to obtain the crystalline form of these salts.

This may explain the fact that only the above named three crystalline salts of Ivabradine are known in the prior art.

It was surprisingly found, however, that few of these new Ivabradine salts can be forced to crystallize. In addition to, it has been surprisingly found that the known hydrobromide and oxalate salts exist in various polymorphic forms, only a few of them possess suitable physico-chemical stability needed for pharmaceutical use.

The amorphous and the hydrate forms of Ivabradine hydrobromide are described in WO 2009 124940 PCT Patent Application.

According to the reproduction of the examples described in this Patent Application it has been found that the obtained Ivabradine hydrobromide is in monohydrate crystalline form.

Surprisingly it was found that recrystallizing the monohydrate in an anhydrous solvent a new anhydrate form is obtained.

The physico-chemical properties of this new anhydrate form are different from the form is described in WO 2009 124940. Its higher aqueous solubility can be advantageous in various technological processes.

The new Ivabradine hydrobromide has a higher melting point and therefore is a more stable crystalline form.

For comparison, the melting point of gamma form of Ivabradine HCl, which is the active pharmaceutical ingredient of the lunched Procoralan drug product, is 110-130 °C. The melting point of Ivabradine hydrobromide is 185 °C.

Although the melting point of the alpha form of Ivabradine hydrochloride is higher (about 195 °C), this form is hygroscopic; it deliquesces at relative humidity above 60 %, and then crystallizes to other solid form.

The Ivabradine salt; according to our invention is not hygroscopic, its crystal structure is stable even at 80 % humidity.

The hydrobromide, salt of Ivabradine of formula (I) according to the invention is a new stable crystalline chemical substance which can be used for economic scale up and for the formulation of the active pharmaceutical ingredients into drug product.

The invention relates to the following process of Ivabradine salts of formula (**I**) the chemical name which of is: 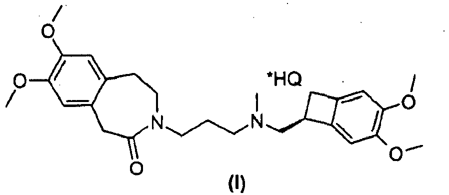 3-{3-[((*S*)-3,4-dimethoxy-bicyclo[4.2.0]octa-1,3,5-triene-7-ilmethyl)-methyl-amino]-propyl}-7,8-dimethoxy-1,3,4,5-tetrahydro-benzo[*d*]azepine-2-one salts (bromide), characterized in that
a) 3-(2-bromo-4,5-dimethoxy-phenyl)-propionitrile of formula (**II**) 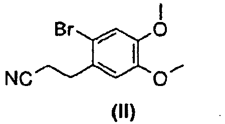 is cyclized with alkali-alkylamide of formula M-NR⁴R⁵ in good yield, wherein R⁴ and R⁵ are hydrogen, C₁-C₄ straight or branched alkyl group, substituted alkyl group respectively or R⁴ and R⁵ are C₄-C₆ respectively alkylidene group which can be unsaturated heterocycle ring. R⁴ and R⁵ together can also be a C₄-C₆ alkylidene group which can form with nitrogen a heterocycle ring and M is alkali metal,
b) the obtained 3,4-dimethoxy-bicyclo[4.2.0]octa-1,3,5-triene-7-carbonitrile compound of formula (**III**) is hydrolyzed, 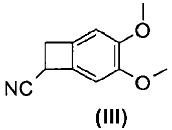
c) the obtained 3,4-dimethoxy-bicyclo[4.2.0]octa-1,3,5-triene-7-carboxylic acid compound of formula (**IV**) 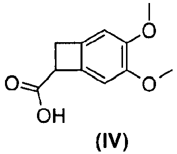 is resolved with suitable chiral base of R¹R²R³N^{*} wherein R¹, R² and R³ are hydrogen, chiral or achiral C₁-C₄ alkyl, substituted chiral or achiral alkyl, chiral or achiral arylalkyl respectively. R¹R²R³N chiral base can also be natural alkaloid or the mixture, the derivatives or the mixture of derivatives thereof.
   From the obtained crystalline diastereomer salt of formula (**Vb**) 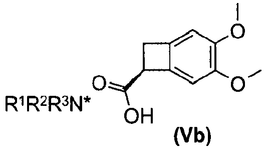 the carboxylic acid of formula (**VIb**) is released or if the diastereomer salt of formula (**Va**) is crystallizes then the carboxylic acid enantiomer of formula (**VIb**) is obtained from the filtrate,
d) the corresponding acid chloride of (*S*)-3,4-dimethoxy-bicyclo[4.2.0]octa-1,3,5-triene-7-carboxylic acid compound of formula (**VIb**) is formed *in situ* and reacted with methylamine without isolation, 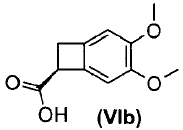
e) the obtained (*S*)-3,4-dimethoxy-bicyclo[4.2.0]octa-1,3,5-triene-7- carboxylic acid - methylamide of formula (**VII**) is reduced 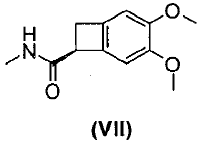 and the ((*S*)-3,4-dimethoxy-bicyclo[4.2.0]octa-1,3,5-triene-7-yl-methyl)-methyl-amine hydrochloride salt of formula (**VIII**) is isolated, 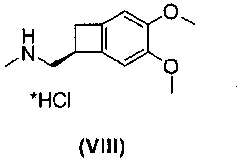
f) The amine hydrochloride of formula (**VIII**) is reacted with 1-(7,8-dimethoxy-1,3-dihydro-2H-3-benzazepine-2-one-3-yl)-3-chloro-propane of formula (**IX**) 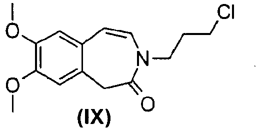 and the obtained 3-{3-[((S)-3,4-dimethoxy-bicyclo [4.2.0] octa-1,3,5-triene-7-ylmethyl)-methyl-amino] -propyl} -7,8-dimethoxy-1,3 -dihydro-2H-3 -benzazepine-2-one of formula (**X**) is isolated 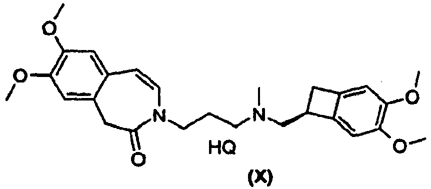 in the form of an acid addition salt of general formula HQ (HQ = NHO₃ or (COOH)₂) and purified by recrystallization.
g) From 3-{3-[((S)-3,4-dimethoxy-bicyclo[4.2.0] octa-1,3,5-triene-7-ylmethyl)-methyl-amino]-propyl}-7,8-dimethoxy-1,3-dihydro-2H-3-benzazepine-2-one salt of formula (X) the base is released with K₂CO₃ with or without isolation,
   the base is subjected to catalytic hydrogenation with Pd-C catalyst in an ethanol or methanol solvent at the pressure of 1-15 bar at temperature of 25-100°C, then the Ivabradine hydrobromide salt of formula (I) is isolated, wherein the crystallization Ivabradine hydrobromide is carried out by cooling the methanol or ethanol solution.
h) advantageously the salt is recrystallized to obtain stable crystalline form thereof.

The reagent of the *step a)* preferably is lithium diisopropyl amide.

In a preferred embodiment of the invention the resolving agent is cinchonine, (*R*)-3,4-dimethoxy-bicyclo [4.2.0.] octa-1,3,5-triene-7-carboxylic acid of formula (**VIa**) is isolated from the filtrate, racemised then the racemate is resolved again.

Preferably 0.2 -1.2 equivalent of chiral base of formula R¹R²R³N is added to the racemic acid of formula (**IV**).

In a more preferred embodiment for the reaction 0.4 - 0.6 equivalent of chiral base of formula R¹R²R³N is used.

In another preferred embodiment of the reaction (S)-3,4-dimethoxy-bicyclo[4.2.0.]octa-1,3,5-triene-7-carboxylic acid methylamide of formula (**VII**) is purified by recrystallization.

In a further preferred embodiment of the reaction 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one of formula (**X**) is purified in the form of oxalate.

The invention also relates to the acid addition salts of 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one of formula (**I**).

HQ = HBr

The process according to the invention consists of the following technological steps:

### Step 1.

3-(2-bromo-4,5-dimethoxy-phenyl)-propionitrile of formula (**II**) (which can be prepared by known methods e.g.: US 4618683) is reacted with alkali-alkylamide of formula M-NR⁴R⁵.

R⁴ and R⁵ are hydrogen, C₁-C₄ straight or branched alkyl group, substituted alkyl group respectively or R⁴ and R⁵ are C₄-C₆ alkylidene group which can be unsaturated heterocycle ring. R⁴ and R⁵ together can also be a C₄-C₆ alkylidene group which can form a heterocycle ring with nitrogen and M is an alkali metal.

Compound of formula (**II**) is reacted e.g. with lithium diisopropyl amide (LDA) and the obtained 3,4-dimethoxy-bicyclo[4.2.0] octa-1,3,5-triene-7-carbonitrile of formula (**III**) is isolated in crystalline form.

### Step 2.

3,4-dimethoxy-bicyclo[4.2.0]octa-1,3,5-triene-7-carbonitrile of formula (**III**) is hydrolyzed by heating with aqueous alkali-metal hydroxide solution and the obtained racemic 3,4-dimethoxy-bicyclo[4.2.0] octa-1,3,5-triene-7-carboxylic acid of formula (**IV**) is isolated in crystalline form.

### Step 3.

Racemic 3,4-dimethoxy-bicyclo[4.2.0]octa-1,3,5-triene-7-carboxylic acid of formula (**IV**) is reacted with the chiral base of general formula of R¹R²R³N wherein R¹, R² and R³ are hydrogen, chiral or achiral C₁-C₄ alkyl, substituted chiral or achiral alkyl, chiral or achiral arylalkyl respectively. R¹R²R³N chiral base can also be natural cinchona alkaloid or the mixture, the derivatives or the mixture of derivatives thereof.

If the crystalline diastereomer salt contains (*S*)-3,4-dimethoxy-bicyclo[4.2.0]octa-1,3,5-triene-7-carboxylic acid of formula (**VIb**) than it is released from a mineral acid in >95% ee enantiomeric purity with or without recrystallization.

This product is reacted further with or without isolation in an organic solvent, preferably in dichloromethane in the next *Step 5.*

If the filtrate of the diastereomer salt formation contains the acid enantiomer of formula (**VIb**) then the mixture of isomers (**VIb**>>**VIa**) is obtained by recrystallization or selective precipitation of the alkali or ammonium salt of the aqueous reaction mixture.

The enantiomer of formula (**VIb**) and the racemic part are separated, the carboxylic acid of formula (**VIb**) is isolated in crystalline form or reacted further without isolation in an organic solvent, preferably in dichloromethane in the next *Step 5.*

### Step 4.

3,4-dimethoxy-bicyclo[4.2.0]octa-1,3,5-triene-7-carboxylic acid of formula (**VIa**) enriched in (*R*)-isomer is obtained by treating the organic filtrates of salt formation and/or that of recrystallization *(Step 3.)* with aqueous acid.

The pure enantiomer of formula (**VIb**) and the isomeric mixture of carboxylic acids of formula (**VIa** + **VIb**) are also isolated..

These mixtures of isomers and the carboxylic acid enriched in (*R*)-isomer are racemized with a base and the racemic carboxylic acid of formula (**IV**) is isolated after mineral acid treatment.

The obtained racemicic carboxylic acid is used in *Step 3*. as starting material. Racemisation of the (*R*)-isomer rich carboxylic acid and the hydrolysos of nitrile of formula (**III**) can be carried out oparalelly in the same reaction mixture as well.

### Step 5.

(*S*)-3,4-dimethoxy-bicyclo[4.2.0]octa-1,3,5-triene-7-carboxylic acid of formula (**VIb**) is reacted with an inorganic or organic acid chloride the *in situ* formed acid chloride is reacted with methylamine in a one-pot reaction.

(*S*)-3,4-dimethoxy-bicyclo[4.2.0]octa-1,3,5-triene-7-carboxylic acid -N-methyl-amide of formula (**VII**) is obtained after crystallization in >99.8% ee purity.

### Step 6.

(*S*)-3,4-dimethoxy-bicyclo[4.2.0]octa-1,3,5-triene-7- carboxylic acid -N-methyl-amide of formula (**VII**) is reduced with a borane complex and ((*S*)-3,4-dimethoxy-bicyclo[4.2.0]octa-1,3,5-triene-7-yl-methyl)-methyl-amine hydrochloride of formula (**VIII**) is obtained. The formed methylamine borane complex is treated with hydrogen chloride in anhydrous solvent. The hydrochloride salt of formula (**VIII**) is purified by recrystallization.

### Step 7.

The amine hydrochloride of formula (**VIII**) is reacted with 1-(7,8-dimethoxy-1,3-dihydro-2H-3-benzazepine-2-one-3-yl)-3-chloro-propane derivatives of formula (**IX**) in the presence of sodium iodide and a base.

The thus obtained3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one is reacted with HNO₃ or (COOH)₂ and the obtained salt of formula (**X**) is isolated. The compound of formula (**X**) is purified by recrystallization.

### Step 8.

3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one salt of formula (**X**) or its free baseform is hydrogenated with Pd-C catalyst in an alcohol type of solvent or acetic acid. Ivabradine salt of formula (**I**) is obtained by treating the reaction mixture with HBr acid which can be purified by recrystallization.

The detailed description of the steps of the invention:

### Step 1.

3-(2-bromo-4,5-dimethoxy-phenyl)-propionitrile of formula (**II**) is reacted with alkali-alkylamide of general formula M-NR⁴R⁵ (R⁴ and R⁵ are hydrogen, C₁-C₄ straight or branched alkyl, substituted alkyl respectively or R⁴ and R⁵ are C₄-C₆ alkylidene group which can be unsaturated heterocycle ring. R⁴ and R⁵ together can also be C₄-C₆ alkylidene group which can form heterocycle ring with the nitrogen and M is an alkali metal) in an aprotic solvent, preferably in tetrahydrofuran (THF) at temperature - 30 - 0 °C, preferably at -15 - 10 °C.

3,4-dimethoxy-bicyclo[4.2.0] octa-1,3,5-triene-7-carbonitrile of formula (**III**) is isolated by crystallization form ethyl acetate, toluol, ethanol, preferably from ethanol.

### Step 2.

3,4-dimethoxy-bicyclo [4.2.0] octa-1,3,5-triene-7-carbonitrile of formula (**III**) is hydrolyzed by heating with aqueous alkali-metal hydroxide solution e.g.: lithium hydroxide, sodium hydroxide, potassium hydroxide, preferably heating with potassium hydroxide in a protic solvent or solvent mixture, preferably in aqueous solution.

The obtained basic solution is acidified with an aqueous mineral acid to pH 1 - 5, preferably with aqueous hydrochloric acid to pH = 3.

The obtained racemic 3,4-dimethoxy-bicyclo[4.2.0] octa-1,3,5-triene-7-carboxylic acid of formula (**IV**) is isolated in crystalline form.

### Step 3.

Racemic 3,4-dimethoxy-bicyclo[4.2.0]octa-1,3,5-triene-7-carboxylic acid of formula (**IV**) is reacted with the chiral base of general formula R¹R²R³N wherein R¹, R² and R³ are hydrogen, chiral or achiral C₁-C₄ alkyl, substituted chiral or achiral alkyl, chiral or achiral arylalkyl respectively. R¹R²R³N chiral base can also be natural cinchona alkaloid or the mixture, the derivatives or the mixture of derivatives thereof, e.g.: cinchonidine, cinchonine, hydrocinchonine and the mixture thereof, (*R*)- or (*S*)-1-naftyl-ethylamine (*S*)-or (*R*)-1-phenylethylamine.

Preferably the reaction is carried out with the mixture of cinchonine and hydrocinchonine in an aprotic solvent, preferably in ethyl acetate and the precipitated crystalline diastereomer salt is isolated.

If the crystalline diastereomer salt contains (*S*)-3,4-dimethoxy-bicyclo[4.2.0]octa-1,3,5-triene-7-carboxylic acid of formula (**VIb**) it is treated with a mineral acid and the formed acid with >95%ee enantiomeric purity is used in the next step (*Step 5*)*.*

If the filtrate of the diastereomer salt formation contains the enantiomer of formula (**VIb**) then the mixture of isomers (**VIb>>VIa**) is isolated and recrystallized or purified by selective precipitation of the alkali or ammonium salt of the aqueous reaction mixture.

The enantiomer of formula (**VIb**) and the racemic part are separated, the carboxylic acid of formula(**VIb**) is isolated in crystalline form or reacted in the next *Step 5.* without isolation. The solvent is an organic solvent, preferably dichloromethane.

### Step 4.

3,4-dimethoxy-bicyclo[4.2.0]octa-1,3,5-triene-7-carboxylic acid of formula (**VIa**) enriched in (*R*)-isomer is obtained by treating the organic filtrates (ethyl acetate solution) of salt formation and recrystallization in *Step 3.* with aqueous acid, preferably with hydrochloric acid.

The crystalline carboxylic acid of formula (**VIa**) is obtained from the organic phase by evaporation and adding an apolar solvent, preferably hexane.

The carboxylic acid is racemized by heating in a protic solvent, preferably in water in the presence of an alkali hydroxide, preferably in the presence of potassium hydroxide.

The racemic carboxylic acid of formula (**IV**) is isolated treating with a mineral acid, preferably with hydrochloric acid to pH 1 - 5, preferably to pH = 3.

The obtained racemic carboxylic acid is resolved again *Step 3..* Racemisation of the (*R*)-isomer rich carboxylic acid and the hydrolysos of nitrile of formula (**III**) can be carried out oparalelly in the same reaction mixture as well.

### Step 5.

(*S*)-3,4-dimethoxy-bicyclo[4.2.0]octa-1,3,5-triene-7- carboxylic acid of formula (**VIb**) is reacted with an inorganic or with organic acid chloride, preferably with oxalyl chloride without catalyst or in the presence of dimethylformamide catalyst in an aprotic solvent, preferably in dichlorometane at temperature 0-80°C, preferably at 0-25°C.

The obtained methylamide of formula (**VII**) is isolated in crystalline form and in desired case is purified by recrystallization.

(*S*)-3,4-dimethoxy-bicyclo[4.2.0]octa-1,3,5-triene-7-carboxylic acid-N-methyl-amide of formula (**VII**) is obtained with >99.8% ee purity and >95% yield.

### Step 6.

(*S*)-3,4-dimethoxy-bicyclo[4.2.0]octa-1,3,5-triene-7-carboxylic acid-N-methyl-amide of formula (**VII**) is reduced with an 1.5-3 mole excess of borane complex, preferably with 2 mole excess of borane-tetrahydrofuran complex at the temperature of 0-70°C, preferably at 20-40°C.

The excess of the borane complex is decomposed in an an alcohol type of reagent e.g.: methanol, ethanol, 2-propanol.

((*S*)-3,4-dimethoxy-bicyclo[4.2.0]octa-1,3,5-triene-7-yl-methyl)-methyl-amine hydrochloride of formula (**VIII**) is obtained after reacting this mixture in anhydrous solvent, preferably in ethyl acetate with hydrogen chloride.

The crystalline hydrochloride salt of formula (**VIII**) is obtained with >88% yield.

### Step 7.

((*S*)-3,4-dimethoxy-bicyclo[4.2.0]octa-1,3,5-triene-7-yl-methyl)-methyl-amine hydrochloride of formula (**VIII**) is alkylated with (1.1 mole equivalent) of 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one derivatives of formula (**IX**) in the presence of 2-5 mole equivalent, preferably 2.5 mole equivalent of sodium iodide and 5-6 mole equivalent of base, preferably 5.5 mole equivalent of potassium carbonate in an anhydrous aprotic solvent, preferably in 1-methyl-2-pyrrolidone (NMP), for 6-18 hours, preferably for 8.5 hours, at 40-80°C, preferably at 60°C.

The obtained crude 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one base is reacted with oxalic acid, or nitric acid and the obtained salt of formula (**X**) is isolated.

The compound of formula (**X**) is purified by recrystallization.

### Step 8.

3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-2-one salt of formula (**X**) or the base thereof deliberated with a base, preferably with K₂CO₃ with or without isolation, preferably without isolation is hydrogenated with Pd-C catalyst in an alcohol type of solvent at the pressure of 1-15 bar, preferably at 10 bar at temperature of 25-100°C, preferably at 45°C.

The obtained Ivabradine salt (hydrobromide) of formula (**I**) is isolated in crystalline form and in desired case it is purified by recrystallization.

The Ivabradine salt of formula (**I**) is obtained usually with >99,5% purity and >75% yield.

The crystallization of the crystalline form of Ivabradine HBr which relates to the invention is carried out by cooling the organic solution, preferably polar organic solution, more preferably methanol or ethanol solution.

The crystallization can also be carried out by precipitation of the organic solution, preferably polar organic solution, more preferably methanol or ethanol with an other solvent which dissolves the compound less.

### The advantages of our process are the followings:

This is a cost effective process using cheap resolution agents and / or they can be regenerated.

The effective enantiomers of the resolved intermediates can be obtained by simple methods.

They can be isolated from the crystalline diastereomer salt or the filtrate of the salt formation depending on the resolution agents.

The efficacy of the resolution step is increased also by racemisation of the carboxylic acid intermediate of the other enantiomers and the regeneration of the racemic intermediate.

The preparation of enantiomer pure ((*S*)-3,4-dimethoxy-bicyclo[4.2.0]octa-1,3,5-triene-7-yl-methyl)-methyl-amine hydrochloride of formula (**VIII**) is solved which is safe and can be used for scale up.

The chemical and enantiomer purity of this intermediate is appropriate for the high quality requirement of the active pharmaceutical ingredients.

The synthesis according to the invention does not need extreme reaction conditions and technological steps, the intermediates can be isolated with simple methods with high yield and purity.

The products of the filtrates of the resolution process are racemizied, the other enantiomers are regenerated, therefore the yield of the process is increased comparing to the known processes.

A method is elaborated to the regeneration of the expensive resolution agents.

According to the above facts the advantages of our process are the followings:
a) A new synthesis with new intermediates in which the intermediates can be purified effectively with simple crystallization methods.
   The active pharmaceutical ingredient can be prepared without extreme reagents and technological conditions with high optical and chemical purity.
b) In the first step of the synthesis 3-(2-bromo-4,5-dimethoxy-phenyl)-propionitrile of formula (**II**) was cyclized by a novel method in an aprotic solvent with alkali-alkylamide at about -10°C and the obtained 3,4-dimethoxy-bicyclo[4.2.0] octa-1,3,5-triene-7-carbonitrile of formula (**III**) is isolated in crystalline form.
   The known cyclization processes are carried out at low temperature (-33°C), using liquid ammonia or extreme flammable sodium amide reagent with high environment pollution risk. The yield of the known processes is much less (60-65%) comparing to the process according to our invention (80-85%).
c) The key intermediate of the synthesis of Ivabradine is ((*S*)-3,4-dimethoxy-bicyclo[4.2.0]octa-1,3,5-triene-7-yl-methyl)-methyl-amine hydrochloride of formula (**VIII**) was prepared by a novel process starting from the optically active carboxylic acid of formula (**VIb**) as a novel intermediate.
   A novel process was elaborated to the resolution of racemic 3,4-dimethoxy-bicyclo[4.2.0]octa-1,3,5-triene-7-carboxylic acid of formula (**IV**).
   (*S*)-3,4-dimethoxy-bicyclo[4.2.0]octa-1,3,5-triene 7-carboxylic acid of formula (**VIb**) was obtained by this process with >95% ee purity.
d) (*R*)-3,4-dimethoxy-bicyclo[4.2.0]octa-1,3,5-triene-7-carboxylic acid of formula (**VIa**) and the isomer mixture was racemized with simple reaction and the racemic acid was resolved again.
   By this method racemic 3,4-dimethoxy-bicyclo[4.2.0]octa-1,3,5-triene-7-carboxylic acid was converted to (*S*)-3,4-dimethoxy-bicyclo[4.2.0]octa-1,3,5-triene-7-carboxylic acid of formula (**VIb**) in high yield.
e) The advantage of the process according to the invention is also that the preparation of the intermediate with high optical purity is not necessary because the methylamide of formula (**VII**) can be purified from enantiomer pollutant effectively with simple recrystallization technology.
f) 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzazepine-one oxalate or nitrate of formula (**X**) are novel intermediates which can be purified effectively with simple recrystallization in high yield.
g) Ivabradine HBr of formula (I) is a novel polymorphic form of a known compound which can be purified effectively with simple recrystallization.
   It can be used for large scale production of active ingredients appropriate for pharmaceutical formulations.

Summarizing, the novel synthesis according to the invention is suitable for cost effective, large scale production of Ivabradine salts.

The purity of the obtained pharmaceutically active ingredient meets the high quality requirements of current standards.

### Examples:

The synthesis according to the invention is illustrated by the following examples:

### Applied measuring conditions:

The optical purity was measured by HPLC method using CHIRALPAK IA (CHIRAL TECHNOLOGIES EUROPE) 250x4.6 mm 5µm HPLC column.

For the identification of the compounds ¹H NMR spectrums were used.

¹H NMR spectrums were obtained on *VnmrS Varian NMR system 400* (ill. *500, 800*) *MHz with cryoprobe (500 and 800 MHz) equipments.*

The chemical shifts are given in ppm (parts per million) compared to tetramethylsilane internal standard.

For the solid phase characterization of the crystalline forms X-Ray Power Diffraction (XRPD), Infrared (IR) and Raman spectroscopic measurements were used.

The X-ray powder diffraction measurements were performed on a PANalytical X'Pert PRO diffractometer using CuKα radiation in reflection geometry with 40 kV accelerating voltage and 40 mA anode current at a scanning rate of 0.013 °, spinning the sample holder by 108 revolution/s.

FT-IR spectra were measured on a Thermo Nicolet 6700 FT-IR spectrometer in KBr pellets accumulating 100 scans at 4 cm⁻¹ spectral resolution.

FT-Raman measurements were performed on a Thermo Nicolet NXR-9650 FT-Raman spectrometer equipped with a Nd:YAG laser operating at 1064 nm equipped with Ge detector with nitrogen cooling. The power of the irradiating beam was set to 300 mW or 500 mW; the applied scan number was 128 or 256 at 4 cm⁻¹ spectral resolution.

The characteristic data reported have to be thought of with the uncertainty accepted by the current state of the art, therefore regarding XRPD measurements "about" means ± 0,2° 2θ uncertainty, and regarding IR and Raman measurements "about" means ± 4 cm⁻¹ uncertainty.

In the examples cyclobutane-carboxylic acid means 3,4-dimethoxy-bicyclo[4.2.0]octa-1,3,5-triene-7-carboxylic acid.

Cyclobutane-carbonitrile means 3,4-dimethoxy-bicyclo[4.2.0]octa-1,3,5-triene-7-carbonitrile.

The chemical name of Ivabradine is 3-{3-[((*S*)-3,4-dimethoxy-bicyclo[4.2.0]octa-1,3,5-triene-7-ylmethyl)-methyl-amino]-propyl}-7,8-dimethoxy-1,3,4,5-tetrahydro-benzo[*d*]azepin-2-one.

Dehydro-Ivabradine means 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-2H-3-benzo[*d*]azepin-2-one.

### Example 1.

### Preparation of 3,4-dimethoxy-bicyclo[4.2.0]octa-1,3, 5-triene-7-carbonitrile (III)

In a 250 ml flask in argon atmosphere 50 ml of anhydrous tetrahydrofuran was added. The mixture was cooled to -10 °C and 33 ml of 2.5 M butyllithium hexane solution was added dropwise while the temperature was kept below -10 °C. Then the reaction was stirred for 10 minutes at -10°C. 13.0 ml of diisopropylamine (or 10.0 ml diethylamine) was added dropwise while the temperature was kept at -10°C.

Then the reaction was stirred for 10 minutes. 10.0 g of 3-(2-bromo-4,5-dimethoxyphenyl)-propionitrile (**II**) dissolved in 30 ml of anhydrous tetrahydrofuran was added dropwise to the solution at -10 °C. The reaction was stirred at -10 °C.

After the reaction had been completed it was allowed to warm up to 0 °C and 100 ml of 1 M hydrochloric acid was added dropwise while the temperature was kept below 20 °C.

After 5 minutes stirring the phases were separated. The aqueous phase was washed twice with 50 ml of toluol. The combined organic phases were washed with 1x50 ml of 1 M hydrochloric acid and 1x50 ml of saturated sodium chloride solution.

The organic phase was evaporated in vacuum to 20 g. 20 ml of ethanol was added to the residue and it was evaporated in vacuum again to 20 g. 30 ml of ethanol was added to the residue and it was evaporated again to 20 g.

The residue was stirred on ice-water. The product was crystallized, it was stirred for 30 minutes at 0-5 °C. Then it was filtered and washed with ethanol. It was dried in vacuum at 40 °C.

5.55 g of the title compound was obtained in 80% yield.

### Example 2.

### Preparation of 3,4-dimethoxy-bicyclo[4.2.0] octa-1,3,5-triene-7-carboxylic acid(IV)

16.4 g (292 mmol) of potassium hydroxide was dissolved in 200 ml of ion exchanged water. 36.78 g (194.4 mmol) of 3,4-dimethoxy-bicyclo[4.2.0]octa-1,3,5-triene-7-carbonitrile of formula (**III**) was added to the potassium hydroxide solution. The mixture was refluxed for 4 hours and then cooled to room temperature.

6.5 g of Aluminium oxide and 0.70 g of charcoal was added to the stirred mixture. It was stirred for 5 minutes, filtered and washed with 75 ml of ion exchanged water.

The filtrate was stirred at room temperature and 27.5 ml (330 mmol) of cc hydrochloric acid was added. The mixture was stirred for 30 minutes at ice-water bath.

The precipitated product was filtered, washed with 500 ml of ion exchanged water at 0-5 °C and dried in vacuum at 55 °C.

38.0 g of the title compound was obtained in 94% yield in the form of white, crystalline, racemic carboxylic acid of formula (**IV**).

### Example 3.

### Preparation of (S)-3,4-dimethoxy-bicyclo[4.2.0]octa-1,3,5-triene-7-carboxylic acid cinchonine salt (Vb, R¹R²R³N*=(+)-cinchonine)

A mixture of 37.8 g of (181.5 mmol) racemic carboxylic acid of formula (**IV**), 380 ml of ethyl acetate and 21.4 g (72.3 mmol) of (+)-cinchonine of 85% purity was refluxed for 15 minutes and then cooled to room temperature. Then the mixture was stirred for 1 hour at 0-5°C and the suspension was filtered.

The crystals were washed with 5x40 ml of cold ethyl acetate, dried in vacuum at room temperature. The filtrate (**Al1**) was regenerated.

25.4 g (13.8 g (66.5 mmol)) of title compound was obtained in 73 % yield (calculated to half of racemate), in the form of white, crystalline (*S*)-cyclobutane-carboxylic acid cinchonine salt. de ≈96 %.

¹H NMR δ(ppm) 6.80(s, 1H); 6.78 (s, 1H); 4.12(m, 1H); 3.71(s, 3H); 3.69(s, 3H); 3.25(dd, 2H); 3.17(dd, 2H);
¹H NMR δ(ppm) 8.85(d, 1H); 8.27(d, 1H); 8.02(d, 1H); 7.74(t, 1H); 7.60(t, 1H); 7.56(d, 1H); 6.08(m, 1H); 5.46(d, 1H); 5.14-5.07(m, 1H); 3.14(m, 1H); 3.09(m, 1H); 2.79(dd, 1H); 2.72(m, 1H); 2.62(m, 1H); 2.26(m, 1H); 1.94(dd, 1H); 1.73(m, 1H); 1.51(m, 1H); 1.37(m, 1H)
¹H NMR δ(ppm) 4.03 (q, 2H); 2.00 (s, 3H); 1.17 (t, 3H)
Mole ratio: (*S*)-3,4-dimethoxy-bicyclo[4.2.0]octa-1,3,5-triene-7-carboxylic acid: Cinchonine : EtOAc = 2 : 1 : 0.4
XRPD reflections measured at: 5.9; 9.4; 10.2; 11.8; 12.6; 13.9; 15.6; 15.8; 17.2; 17.8; 18.8; 19.2; 20.4; 21.4; 22.5; 25.2; 25.9 and 27.1 °2θ.
IR absorption bands measured at: 3080; 2995; 2930; 2833; 1738; 1712; 1591; 1564; 1485; 1466; 1312; 1206; 1179; 1154; 1071; 984; 935; 851 and 759 cm⁻¹.

### Example 4.

### Preparation of (S)-3,4-dimethoxy-bicyclo[4.2.0]octa-1,3,5-triene-7-carboxylic acid (VIb) from its cinchonine salt (Vb, R¹R²R³N*=(+)-Cinchonine)

25.5 g of (*S*)-3,4-dimethoxy-bicyclo[4.2.0]octa-1,3,5-triene-7-carboxylic acid cinchonine salt *(**Vb,** R¹R²R³N**=*(*+*)-Cinchonine)* was suspended in 125 ml of dichlorometane.

40 ml of 3 M hydrochloric acid was added, it was stirred for 5 minutes intensively and then the phases were separated.

The organic phase was washed with 20 ml of 3 M hydrochloric acid. The combined aqueous phases were washed with 3x25 ml of dichlorometane.

The organic phases were combined, dried with sodium sulphate, filtered and evaporated.

13.81 g (*S*)-carboxylic acid of title compound *(**VIb**)* was obtained in the form of white crystals, ee: 96 %. [α]_{D}: +85.2 (20 °C, c=1, 1 M NaOH)

***¹H-NMR (ppm)*** V-400 (¹H: 400MHz, DMSO-d₆): 13.01-11.68 br (1H); 6.81 s (1H); 6.78 s (1H); 4.13 dd (1H); 3.71 s (6H); 3.27 dd (1H); 3.17 dd (1H)

### Example 5.

### Preparation of (S)-3,4-dimethoxy-bicyclo[4.2.0]octa-1,3,5-triene-7-carboxylic acid(S)-(-)-1-naphthyl-ethylamine salt (Vb, R¹R²R³N*= (S)-(-)-1-naphthyl-ethylamine)

1.46 g of (7.02 mmol) of the racemic carboxylic acid of formula (**IV**) was dissolved in 16 ml of ethyl acetate with heating and 1.602 g of (3.51 mmol, 0.5 equiv.) (*S*)-(-)-1-naphthyl-ethylamine was added.

The crystal suspension was stirred further 1 hour on water bath of 18 °C, then it was filtered, washed with ethyl acetate (3x2 ml) and dried.

0.88 g (66 % calculated to half of racemate), ee: ≈93 % of the title compound was obtained.

Recrystallization of the salt: 0.68 g (1.69 mmol) of diastereomer salt was refluxed in 13.4 ml of ethyl acetate with intensive stirring, then it was cooled, filtered and washed with ethyl acetate (3x2 ml).

0.54 g of title compound was obtained in 79 % yield with ee: 99,3 %.
¹H NMR (CDCl₃, 500 MHz): 7.99 (1H, d, J 8.5), 7.86 (1H, d, J 8.0), 7.76 (1H, d, J 8,0), 7.60 (1H, d, J 7,0), 7.53 (1H, t, J 7.0), 7.48 (1H, t, J 7.0), 7.41 (1H, t, J 8.0), 6.61 (1H,s), 6.59 (1H, s), 5.11 (3H, br.s), 4.98 (1H, q, J 6,5), 3.97 (1H, m), 3.78 (3H, s), 3.71 (3H, s), 3.12 (2H, m), 1.55 (3H, d, J 6,5).
[α]_{D}: +15.1 (c 0,6, EtOH)
XRPD reflections measured at: 5.3; 10.1; 10.6; 15.9; 16.6; 17.7; 17.8; 18.7; 19.9; 21.2; 23.8; 26.6 and 30.1 °2θ.
IR absorption bands measured at: 3070; 2994; 2933; 2834; 1629; 1571; 1522; 1485; 1391; 1380; 1315; 1206; 1153; 1074; 1011; 991; 854; 838; 804; 779; 740; 613; 439 and 413 cm⁻¹.

### Example 6.

### Preparation of (S)-3,4-dimethoxy-bicyclo[4.2.0]octa-1,3,5-triene-7-carboxylic acid (VIb) from (S)-(-)-1-naphthyl-ethylamine salt

0.54 g (***Vb,** R¹R²R³N**= *(S)-(-)-1-naphthyl-ethylamine)* salt obtained in *Example 5*. was dissolved in the mixture of 8.1 ml of dichloromethane and 5.2 ml of water and 1.62 ml of 5 M hydrochloric acid was added.

It was stirred for 20 minutes intensively and the phases were separated. The aqueous phase was extracted with dichloromethane (3x2 ml), dried with sodium sulphate and evaporated.

The residue is (*S*)-3,4-dimethoxy-bicyclo[4.2.0]octa-1,3,5-triene-7-carboxylic acid (**VIb**) in the form of white solid.

0.30 g (ee: 99.3 %, calculated to half of racemate) of the title compound was obtained in 56 % yield
[α]_{D}: +61,4 (c:1, MeOH), Op.: 136-142 °C.

From the filtrate further 0.07 g, ee: 20 %, [α]_{D}: +11.5 (c:1, MeOH) of carboxylic acid *(**VIb**)* was obtained.

### Example 7.

### Preparation of (R)-3,4-dimethoxy-bicyclo[4.2.0]octa-1,3,5-triene-7-carboxylic acid (VIa) from (S)-(-)-1-naphthyl-ethylamine salt

The filtrate of (*S*)-(-)-1-naphthyl-ethylamine salt obtained in *Example 5.* was treated according to *Example 6.*

0.99 g (134% calculated to half of racemate) of the title compound was obtained in 56 % yield with ee: 48.0 %. [α]_{D}: -29.5 (c:1, MeOH).

The obtained cyclobutane carboxylic acid was racemized according to *Example 12.*

### Example 8.

### Preparation of (S)-3,4-dimethoxy-bicyclo [4.2.0]octa-1,3,5-triene-7-carboxylic acid (R)-(+)-1-phenyl-ethylamine salt (Vb, R¹R²R³N*= (R)-(+)-1-phenyl-ethylamine)

0.624 g (3.0 mmol) of racemic carboxylic acid of formula **(IV)** was dissolved in 6 ml of ethyl acetate with heating and 0.145 g (1.2 mmol, 0.4 equivalent) of (*R*)-(+)-1-phenylethylamine was added.

Then the reaction mixture was seeded and cooled. The crystal suspension was stirred for further 2 hours, filtered, washed with ethyl acetate (3 x 0.5 ml) and dried.

0.18 g ((46 % calculated to 0.4 equiv resolution agent) diastereomer salt was obtained with ≈92,5 % ee purity.

Recrystallization of the salt: 0.17 g (0.52 mmol) of diastereomer salt was refluxed in ethyl acetate and it was cooled, stirred for 30 minutes at room temperature, filtered and washed with ethyl acetate (3x0.2 ml).

0.13 g of crystalline diastereomer salt was obtained in 70 % yield with ee: 98.3 % purity.
¹H NMR (CDCl₃, 500 MHz): 7.26 (5H, m), 6.65 (1H, s), 6.62 (1H, s), 5.40 (3H br.s), 4.03 (1H, q, J 6,5), 3.96 (1H, m), 3.82 (3H, s), 3.76 (3H, s), 3.18 (1H, dd, J 13.5, 5.0), 3.11 (1H, d, J 13.5), 1.39 (3H, d, J 6.5). [α]_{D}: +36.8 (c 0.6, EtOH).
XRPD reflections measured at: 9.9; 11.2; 12.3; 13.2; 17.8; 18.5; 18.7; 19.0; 19.7; 20.9; 22.8; 23.5; 24.1; 25.7 and 28.4 °2θ.
IR absorption bands measured at: 3060; 2999; 2980; 2928; 2869; 2829; 2640; 2240; 1648; 1557; 1519; 1485; 1465; 1451; 1396; 1378; 1309; 1206; 1090; 1069; 1012; 984; 852; 843; 776; 755; 710; 560; 545 and 482 cm⁻¹.

The filtrate of diastereomer salt was treated according to *Examples 6. and 7.*

### Example 9.

### Preparation of (R)-3,4-dimethoxy-bicyclo[4.2.0]locta-1,3,5-triene-7-carboxylic acid cinchonidine salt (Va, R¹R²R³N*= cinchonidine)

20.0 g (96 mmol) of racemic cyclobutane carboxylic acid of formula **(IV)** and 4.10 g (48 mmol) cinchonidine was dissolved in 330 ml acetone under reflux.

The solution was crystallized with cooling.

The suspension was stirred for 30 minutes, filtered and washed with cool acetone. The product was dried in vacuum.

18.43 g. of the title compound was obtained in 89 % yield.

The product was recrystallized from 200 ml of hot methyl-isobutyl-ketone to give 17.58 g of the title compound.

*¹**H NMR (ppm)*** (¹H: 500MHz, DMSO-d₆): 8.86 d (1H); 8.34 d (1H); 8.04 d (1H); 7.74 m (1H); 7.62-7.56 m (2H); 6.79 s (2H); 6.15-4.45 br (1H); 5.83 m (1H); 5.50 d (1H); 5.00 d (1H); 4.93 d (1H); 4.12 dd (1H); 3.71 s (3H); 3.69 s (3H); 3.37 m (1H); 3.27-3.14 m (3H); 2.98 dd (1H); 2.62-2.49 m (2H); 2.33-2.26 m (1.4H); 2.06 s (0.66H); 2.00 m (0.22H); 1.81-1.61 m (4H); 1.50 m (1H); 0.85 m (1.32H)
Mole ratio: (*R*)-3,4-dimethoxy-bicyclo[4.2.0]octa-1,3,5-triene-7-carboxylic acid: cinchonidine : Methyl-isobutyl-ketone = 1 : 1 : 0,22
XRPD reflections measured at: 6.0; 6.2; 7.4; 11.9; 16.3; 17.1; 17.9; 18.8; 19.2; 20.1; 21.1; 23.6 and 24.7 °2θ.
IR absorption bands measured at: 3069; 2998; 2931; 2831; 1711; 1593; 1509; 1485; 1465; 1383; 1310; 1207; 1176; 1099; 1070; 1007; 986; 856; 810; 784; 757; 646; 633 and 615 cm⁻¹.

The filtrate was evaporated and dissolved in 150 ml of ethyl acetate. 50 ml of distilled water and 10% hydrochloric acid was added adjusting the pH to 3.

The phases were stirred for 5 minutes and then separated. The aqueous phase was washed with 50 ml of ethyl acetate.

The organic phases were combined,dried and evaporated.

11.57 g of (*S*)-carboxylic acid of formula ***(VIb)*** was obtained with ee.: 58 % purity.

### Example 10.

### Enantiomer enrichment by recrystallization of (S)-3,4-dimethoxy-bicyclo[4.2.0]octa-1,3,5-triene-7-carboxylic acid (VIb)

The optically active carboxylic acid of formula *(**VIb**)* was dissolved in warm ethanol and cooled. The precipitated crystalline product was stirred for 1 hour, filtered at about 27 °C, washed with ethanol and dried.

Starting from low or medium ee purity samples the crystalline carboxylic acid of formula (**VIb**) has lower ee purity and the mother liquior has higher ee purity.

Starting form high ee purity samples the optically pure product is crystallized and the less pure product remains in the filtrate.

| Starting *(VIb)* carboxylic acid | | Ethanol (ml) | Crystalline *(**VIb**)* carboxylic acid | |
|---|---|---|---|---|
| Weight | ee₀ | | Yield | ee |
| 0.38 g | 48.4 % | 1.0 | 53 % | 34 % |
| 0.15 g | 95.0 % | 0.4 | 43 % | 100 % |

### Example 11.

### Enantiomer enrichment by selective precipitation of (S)-3,4-dimethoxy-bicyclo[4.2.0]octa-1,3,5-triene-7-carboxylic acid (VIb)

Enantiomer mixture containing carboxylic acid of formula (**VIb > VIa**) was suspended in tenfold water and equivalent amount of 1 mol/l sodium hydroxide solution was added.

0.5 equivalent of hydrochloric acid was added (except for the last case when first 90 % of hydrochloric acid was added).

The precipitated product was stirred for 20 minutes, filtered, washed with water, dried (product I.).

The filtrate was further acidified with hydrochloric acid and the precipitated fraction was filtered, washed with water, dried (product II.).

The ee values of these fractions were mesured by HPLC. The following results were obtained starting form carboxylic acid of formula (**VIb**) (ee₀).

| Starting *(**VIb**)* carboxylic acid | Product I. | | Product II. | |
|---|---|---|---|---|
| ee₀ | Yield | ee_{I} | Yield | ee_{II} |
| 30 % | 19 % | 6 % | 59 % | 49 % |
| 42 % | 60 % | 28 % | 35 % | 70 % |
| 47 % | 58 % | 25 % | 30 % | 75 % |
| 70 % | 52 % | 72 % | 28 % | 62 % |
| 87 % | 89 % | 93 % | 6 % | 72 % |

### Example 12.

### Racemization of (R)-3,4-dimethoxy-bicyclo[4.2.0]octa-1,3,5-triene-7-carboxylic acid (VIa)

The filtrate (**Al1**) obtained in *Example 3.* was washed with 30 ml of 10 weight % hydrochloric acid, then three times with 15 ml of 10 weight % hydrochloric acid.

The organic phase was washed three times with 15 ml of ion exchanged water, then 20 ml of saturated NaCl solution.

The organic phase was evaporated in vacuum to crystal suspension and it was diluted with hexane.

The suspension was stirred for 1 hour on ice-water bath, then it was filtered and washed with the mixture of 15 ml hexane : ethyl acetate = 2 : 1.

The mixture was stirred for 1 hour, the precipitated crystals were filtered and dried.

19.67 g (52 % calculated to racemate) carboxylic acid of formula **(VIa)** was obtained in the form of white crystals.

7.96 g (142 mmol) of potassium hydroxide was dissolved in 97 ml of ion exchanged water. 19.66 g (94.4 mmol) of (*R*)-carboxylic acid of formula **(VIa)** obtained in this *Example* was added to the aqueous base.

The reaction mixture was stirred for 4 hours. The racemization was followed by chiral HPLC method.

The mixture was cooled to room temperature and 13.3 ml (15.9 g, 160 mmol) of cc. hydrochloric acid was added.

The obtained white product was stirred for 30 minutes on ice-water bath. The precipitated crystals were filtered, washed with 243 ml of 0-5 °C ion exchanged water and dried at 55 °C in vacuum.

18.5 g (94%) of racemic carboxylic acid of formula **(IV)** was obtained in the form of white crystals.

### Example 13.

### Preparation of (S)-3,4-dimethoxy-bicyclo[4.2.0]octa-1,3,5-triene-7-carboxylic acid-N-methyl-amid (VII)

### Preparation of (S)-3,4-dimethoxy-bicyclo[4.2.0]octa-1,3,5-triene-7-carboxylic acid (VIb) from cinchonine salt (Vb, R¹R²R³N*=(+)-cinchonine)

25.5 g of *(S)-3,4-dimethoxy-bicyclo[4.2.0]octa-1,3,5-triene-7-carboxylic* acid cinchonine salt (**Vb**) (containing about 66.7 mmol carboxylic acid of formula (**VIb**). Purity:>95% de) was suspended in the mixture of 125 ml of dichlorometane and 40 ml of 3M HCl (120 mmol) solution.

The mixture was stirred until two clear phases were obtained. The phases were separated, the organic phase was washed with 1x20 ml of 3M (60 mmol) HCl solution.

The combined aqueous phase was extracted with 3x25 ml dichloromethane. The combined organic phase was dried on sodium sulphate, filtered and washed with 3x15 ml of dichloromethane, evaporated in vacuum and dried.

13.7g (65.8 mmol) of title compound was obtained.

### Acid chloride and amide (VII) formation

13.5 g (64.8 mmol) (*S*)-3,4-dimethoxy-bicyclo[4.2.0]octa-1,3,5-triene-7-carboxylic acid of formula (**VIb**) was suspended in 70 ml of dichloromethane and 0.1 ml of dimethylformamide was added.

The suspension was stirred at 15-25°C and the solution of 5.8 ml (67.6 mmol) oxalylchloride and 20 ml of dichloromethane was added in 15-30 minutes at 25°C.

After the reaction had been completed, the mixture was added to a stirred mixture of 25 ml of 40% aqueous solution of methylamine and 140 ml of dichloromethane at 0-10°C.

After the reaction had been completed the reaction mixture was heated to 20-25°C and diluted with 25 ml of water.

The phases were separated and the aqueous phase was washed with 1x25 ml of dichloromethane.

The combined organic phase was evaporated to 80 g weight and 80 ml n-hexane was added. The suspension was stirred at 0-10°C for 30 minutes, filtered and washed with the mixture of 1x10 ml of dichloromethane - n-hexane 1:1. It was dried at 25°C in vacuum.

13.4 g (93%), enantiomer purity: >99.8% ee (chiral HPLC) of the title product was obtained.
*¹**H-NMR (ppm)*** V-400 (¹H: 400MHz, DMSO-d₆): 8.66br (0.15H); 7.80 br (1H); 6.81 s (1H); 6.78 s (1H); 4.01 dd (1H); 3.70 s (6H); 3.10-3.22 m (2H); 2.61 d (0.66H); 2.66 d (3H)

### Example 14.

### Preparation of ((S)-3,4-dimethoxy-bicyclo[4.2.0]octa-1,3,5-triene-7-yl-methyl)-methyl-amine hydrochloride (VIII)

13.0 g (58.8 mmol) *(S)-3,4-dimethoxy-bicyclo[4.2.0]octa-1,3,5-triene-7-carboxylic* acid-N-methyl-amide of formula *(**VII**)* was suspended in 70 ml of anhydrous tetrahydrofurane and 120 ml of 1M borane-tetrahydrofurane solution was added.

The reaction mixture was stirred at 20-25°C until clear solution was obtained and then it was stirred at 40-50°C.

After the reaction had been completed the solution was cooled to 0-5°C and 20 ml of methanol was added.

The reaction mixture was stirred for further 15 minutes and then 20 ml of 20% hydrogen chloride solution in anhydrous ethyl acetate was added and it was refluxed for 2 hours. Then the mixture was cooled to 0-5°C, stirred for 30 minutes at this temperature, filtered and washed with 3x10ml of cold tetrahydrofurane. It was dried in vacuum at 25°C.

12.5 g (88%) of title compound was obtained.

*¹**H-NMR:*** V-800 (¹H: 800MHz, DMSO-d₆): 9.14 br (1H); 6.99 s (1H); 6.80 s (1H); 3.72-3.70 m (7H); 3.28-3.21 m (2H); 3.08 m (1H); 2.94 dd (1H); 2.58 (3H)

### 15. Example

### Preparation of 3-{3-[((S)-3,4-dimethoxy-bicyclo[4.2.0]octa-1,3, 5-triene-7-ylmethyl)-methylamino]-propyl}-7,8-dimethoxy-1,3-dihydro-benzo[d]azepine-2-one oxalate (X, HQ=(COOH)₂)

In 50 ml flask in nitrogen atmosphere 10 ml of absolute 1-methyl-2-pirrolidinone (NMP) and 1.0 g (3.3 mmol) of chloropropyl-dihydro-benzazepinone derivative of formula **(IX)** was added.

1.2 g (8.25 mmol) of sodium iodide, 2.28 g (16.5 mmol) of K₂CO₃, 0.73 g of (3.0 mmol) of ((*S*)-3,4-dimethoxy-bicyclo[4.2.0]octa-1,3,5-triene-7-yl-methyl)-methyl-amine hydrochloride of formula (**VIII**) was added in stirring at room temperature.

It was stirred at 60°C. After the reaction had been completed the mixture was cooled to room temperature and 40 ml of distilled water and 30 ml of ethyl acetate was added.

The phases were separated; the aqueous phase was extracted again with 30 ml of ethyl acetate.

The combined organic phase was stirred with 20 ml of 3 mol/l HCl solution, then the phases were separated.

20 ml of 4 mol/l NaOH solution was added to the aqueous-acidic phase and it was extracted with ethyl acetate.

The combined organic phase was washed with 20 ml of water, dried, filtered and it was evaporated to 30 ml residue.

To the residue 0.27 g (3 mmol) anhydrous oxalic acid solved in 3 ml of methanol was added at room temperature.

The suspension was stirred for 2 hours at room temperature, filtered, the crystals were washed with 2x5 ml of ethyl acetate and dried in vacuum.

1.3 g (76%) of title compound was obtained.

### Recrystallization:

1.2 g (2.2 mmol) of 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-benzo[d]azepine-2-one oxalate of formula (**X,** HQ=(COOH)₂) obtained in previous step was dissolved at 80°C in 30 ml of ethanol.

The mixture was cooled to room temperature and stirred for 1 hour, filtered, washed with 2x30 ml of ethanol and dried in vacuum.

1.1 g (83%) of the recrystallized product was obtained in dihydrate form, the water content thereof by TG measurement was 5.9 %.
XRPD reflections measured at: 4.1; 8.2; 10.8; 12.3; 14.0; 14.6; 16.2; 16.5; 17.6; 17.9; 16.8; 20.6; 21.4; 21.6; 22.6; 23.8; 24.8 and 25.7 °2θ.
IR absorption bands measured at: 3634; 3574; 3010; 2949; 2924; 2841; 1726; 1653; 1516; 1484; 1466; 1418; 1407; 1314; 1274; 1210; 1099; 1072; 1050; 1033; 990; 831; 781 and 502 cm⁻¹

### Example 16. (Reference Example not belonging to the invention)

### Preparation of Ivabradine hydrochloride (I, HQ=HCl)

10.0 g of 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-benzo[d]azepin-2-on oxalate *(**X**, HQ=(COOH)₂)* was added to 75ml of 10% K₂CO₃ solution. The mixture was stirred for 5 minutes, then 75 ml of ethyl acetate was added. The mixture was stirred for 5 minutes and the phases were separated.

The organic phase was washed with 2x50 ml distilled water and evaporated to 20 g residue.

To the residue 100 ml of methanol was added and it was evaporated again to 40 g of weight.

To the residue 1.49 ml of 37% HCl-solution was added, it was stirred in argon atmosphere and 0.84 g of 10 % Pd/C was added.

The reaction mixture was stirred in a hydrogen reactor for 6 hours at 45°C in H₂-atmosphere at 10 bar pressure.

After the reaction had been completed the mixture was filtered, the obtained clear solution was evaporated in vacuum to 23 g.

To the residue 130 ml of acetonitrile was added and concentrated again to 70 g of weight.

The precipitated crystal suspension was cooled to 0°C, stirred for 1 hour and filtered. The crystals were washed with 2×5 ml of acetonitrile at 0°C.

To these crude crystals 215 ml of acetonitrile was added and it was refluxed. The hot mixture was filtered and cooled to room temperature.

The precipitated white crystals were cooled to 0°C, stirred for 1 hour, filtered and washed with acetonitrile at 0°C and dried in vacuum at 40°C.

7.03 g (77.5%) of the title product was obtained.

### Example 17. (Reference Example not belonging to the invention)

### Preparation of Ivabradine nitrate (I, HQ=HNO₃) (Form I)

4.00 g (8.54 mmol) of Ivabradine base was dissolved in 75 ml of ethanol and 5.17 ml of 104 g/l aqueous nitric acid (8.54 mmol) was added.

The solution was evaporated in vacuum and 50 ml of ethanol was added to the residue. The solution was evaporated again until the product precipitated. Finally the suspension was evaporated to 23 g. The Ivabradine nitrate salt suspension was stirred for 30 minutes at 0-5 °C, filtered and washed with cold ethanol.

4.16 g (92%) of the title product was obtained in the form of white crystals.
***¹H-NMR***(DMSO-d₆, 400 MHz): 1.93 br (2H); 2.85 br (4H); 3.00 m (2H); 3.07 br (2H); 3.21-3.34 ol (2H); 3.42 m (2H); 3.53 br (1H); 3.62-3.73 m (13H); 3.74-3.85 m (4H); 6.68 s (1H); 6.72 s (1H); 6.81 s (1H); 6.86 br (1H); 9.32 br (1H).
XRPD reflections measured at: 5.2; 7.7; 10.3; 11.7; 14.3; 15.0; 16.1; 17.5; 17.8; 18.0; 18.5; 19.2; 20.7; 22.4; 23.2; 25.9 and 26.8 °2θ.
IR absorption bands measured at: 3001; 2917; 2833; 1656; 1522; 1486; 1464; 1441; 1384; 1303; 1249; 1219; 1186; 1165; 1104; 1063; 1003; 882; 844 and 827 cm⁻¹-nél.
Raman absorption bands measured at: 3070; 2999; 2934; 2918; 2893; 1647; 1607; 1589; 1485; 1442; 1430; 1357; 1318; 1185; 1040; 814; 775; 745; 725; 699 and 507 cm⁻¹

### Example 18. (Reference Example not belonging to the invention)

### Recrystallization of Ivabradine nitrate (I, HQ=HNO₃) (Form II)

0.30 g of Ivabradine nitrate (**I,** HQ=HNO₃) obtained *in Example* 17. was suspended in the mixture of 2 ml of ethanol:water=10:1 and it was stirred for a week at 25 °C. Then the suspension was filtered and dried in vacuum.
XRPD reflections measured at: 14.2; 15.6; 16.5; 16.7; 17.7; 18.0; 18.6; 20.0; 22.0; 22.3; 22.9; 23.6; 25.6 and 29.7 °2θ.
IR absorption bands measured at: 3016; 2969; 2941; 2908; 2763; 1613; 1522; 1489; 1467; 1347; 1307; 1250; 1224; 1209; 1183; 1161; 1108; 1060; 1027; 1008; 968; 845; 831; 577 and 509 cm⁻¹.
Raman absorption bands measured at: 3081; 3017; 2969; 2920; 2908; 1609; 1591; 1468; 1449; 1424; 1361; 1313; 1277; 1043; 802; 778; 745; 727; 717; 697 and 486 cm⁻¹

### Example 19.

### Preparation of Ivabradine hydrobromide (I, HQ=HBr)

3.00 g (6.17 mmol) of Ivabradine base was dissolved in 32 ml of ethyl acetate and 0.69 ml (1.02 g solution, 497 mg HBr, 6.15 mmol) 48,7% of HBr aqueous solution was added.
The reaction mixture was heated to reflux temperature and then it was cooled to room temperature.

The suspension was diluted with 256 ml of ethyl acetate and stirred for overnight at room temperature. The crystals were filtered and dried in vacuum.
3.2 g (91%) of the title compound was obtained.

The obtained Ivabradine HBr salt was recrystallized from methanol:ethyl acetate.

1.17 g of the salt was dissolved in 4.5 ml of methanol at 60 °C, then it was cooled to 40 °C, and 8 ml of ethyl acetate was added.

Then the solution was cooled to 25 °C and 32 ml of ethyl acetate was added. The suspension was stirred at 25 °C for 1 hour and then it was cooled to 0 °C and stirred for further 1 hour.

The crystals were filtered and dried in vacuum. 1.05 g of title product was obtained.
XRPD reflections measured at: 5.0; 12.5; 14.8; 15.2; 15.9; 17.3; 17.5; 17.8; 18.5; 20.0; 20.4; 21.8; 22.6; 23.8; 25.0; 29.7 and 35.1 °2θ.
IR absorption bands measured at: 2999; 2930; 2833; 2697; 2643; 2610; 1664; 1522; 1484; 1463; 1418; 1304; 1250; 1219; 1171; 1104; 1062; 1003; 878 and 845 cm⁻¹.
Raman absorption bands measured at: 3064; 3012; 2998; 2956; 2948; 2918; 2909; 2891; 1654; 1607; 1588; 1483; 1448; 1431; 1422; 1317; 1190; 1004; 811; 769; 743; 727; 701 and 505 cm⁻¹

### Example 20. (Reference Example not belonging to the invention)

### Preparation of Ivabradine oxalate (I, HQ-(COOH)₂)

0.94 g (0.002 mole) of Ivabradine base was dissolved in 5 ml of ethyl acetate at room temperature by stirring, and 0.18 g (0.002 mole) of anhydrous oxalic acid of 1 ml methanol solution was added. The oxalate salt was precipitated, stirred for 1 hour, then the product was filtered and washed with 1 ml of ethyl acetate and dried on air.

1.02 g (91%) of Ivabradine oxalate was obtained. This product was recrystallized from the mixture of acetone and water.

1.0 g of salt was dissolved in the mixture of 15 ml of acetone:water=10:1 at 60 °C. The solution was cooled to 25 °C and 15 ml of acetone was added.

The suspension was stirred 1 hour at 25 °C and further 1 hour at 0 °C. The crystals were filtered and dried in vacuum.

0.8 g of the title compound was obtained.
XRPD reflections measured at: 4.7; 7.5; 8.0; 9.2; 10.2; 11.7; 11.9; 12.2; 12.8; 15.1; 15.8; 16.8; 17.3; 17.9; 19.0; 19.3; 20.2; 21.1; 22.1; 22.7; 23.2; 23.8; 24.6 and 27.7 °2θ.
IR absorption bands measured at: 3447; 2961; 2937; 2835; 2692; 1718; 1644; 1521; 1486; 1464; 1406; 1304; 1279; 1249; 1224; 1210; 1184; 1108; 1070; 1031; 1003; 832; 720; 700; 499 and 474 cm⁻¹.

### Example 21. (Reference Example not belonging to the invention)

### Preparation of dehydro-Ivabradine nitrate (X, HQ=HNO₃)

495 mg (1.02 mmol) of dehydro-Ivabradine base was dissolved in 3 ml of ethanol and 0.63 ml of 104 g/dm³ aqueous nitric acid was added. The solution was evaporated in vacuum and the residue was diluted with 5 ml of ethanol. The solution was evaporated. The solvent exchange was repeated until crystallization.

The crystal suspension was stirred for overnight at room temperature and 1 hour at 0-5 °C.

The crystals were filtered and washed with cold ethanol.

313 mg (56 %) of the title compound was obtained.
***¹H-NMR*** (DMSO-d₆, 800 MHz): 1.86 br (2H); 2.79 br (4H); 2.97 br (2H); 3.14m(1H); 3.21m(1H), 3.39 s (2H) 3.52 br (2H); 3.62 m (2H); 3.69-3.72 m (9H); 3.74 s (3H); 6.44 d (1H); 6.45 d (1H); 6.78 s (1H); 6.81 br (1H); 6.88 s (1H); 6.93 s (1H); 9.25 br (1H).
XRPD reflections measured at: 5.2; 7.8; 10.3; 11.5; 14.1; 15.4; 15.9; 17.7; 18.1; 18.8; 19.8; 20.7; 21.9; 22.2; 22.9; 23.4; 24.9 and 27.8 °2θ.
IR absorption bands measured at: 3000; 2949; 2917; 2836; 1661; 1516; 1486; 1466; 1440; 1403; 1384; 1303; 1277; 1215; 1189; 1117; 1097; 1062; 993; 884; 841; 775 and 756 cm⁻¹.

### 22. Example (Reference Example not belonging to the invention)

### Preparation of Ivabradine hydroiodide salt (1, HQ=HI)

546 mg (1.12 mmol) of Ivabradine base was dissolved in 6 ml of hot ethanol. 0.155 ml of 57% aqueous hydrogen iodide solution was added.

The solution was cooled and the product was crystallized. The suspension was stirred for 30 minutes at 0-5 °C.

The crystals were filtered and washed with ethanol.

450 mg (65 %) of title compound was obtained.
XRPD reflections measured at: 5.0; 12.5; 14.4; 15.3; 16.3; 17.5; 17.8; 18.5; 19.2; 20.0; 21.8; 22.8; 23.2; 23.9; 25.1; 27.1; 29.2; 31.9 and 35.7 °2θ.
IR absorption bands measured at: 2998; 2922; 2830; 2724; 2644; 1660; 1521; 1485; 1463; 1439; 1318; 1303; 1249; 1218; 1105; 1071; 1059; 1000; 880; 841; 578 and 505 cm⁻¹.
Raman absorption bands measured at: 3067; 3000; 2956; 2923; 2907; 2893; 2724; 1652; 1606; 1589; 1441; 1430; 1422; 1319; 1184; 967; 812; 744; 724; 699; 505; 472 and 376 cm⁻¹.

### Example 23. (Reference Example not belonging to the invention)

### Preparation of Ivabradine perchlorate salt (I, HQ=HClO₄)

0.92 g (0.002 mole) of Ivabradine base was dissolved in 4 ml of ethanol and 0.2 ml (0.002 mole) of 60% perchloric acid was added during stirring at room temperature.

The perchlorate salt was precipitated, the ethanol solvent was removed and the sticky solid residue was triturated with 20 ml of diisopropyl ether, filtered and dried on air.

0.96 g of title compound was obtained.

The obtained 0.96 g of salt was suspended in 10 ml of ethanol. The mixture was heated to 90 °C and it was cooled and stirred for 4 hours.

Then it was filtered and dried in vacuum.

0.90 g of title compound was obtained.
XRPD reflections measured at: 5.1; 9.3; 10.2; 11.7; 12.5; 13.4; 14.1; 14.3; 15.0; 15.4; 16.0; 17.3; 17.8; 18.5; 18.9; 20.1; 21.1; 21.9; 22.4; 22.6; 23.1; 25.2 and 26.5 °2θ.
IR absorption bands measured at: 3125; 2997; 2940; 2919; 2833; 1657; 1521; 1485; 1463; 1439; 1318; 1304; 1250; 1222; 1105; 1002; 880; 625; 577 and 506 cm⁻¹.
Raman absorption bands measured at: 3073; 2999; 2955; 2935; 2919; 1648; 1606; 1589; 1482; 1441; 1430; 1319; 1184; 930; 812; 772; 744; 723; 697; 625 and 545 cm⁻¹.

## Claims

1. Process for the synthesis of Ivabradine hydrobromide of formula **(I)** with HQ = HBr, 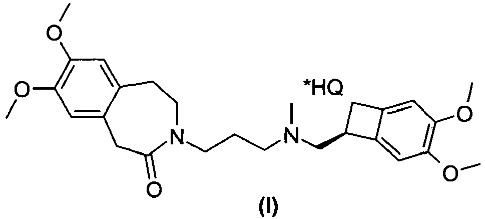 the chemical name of which is: 3-{3-[((*S*)-3,4-dimethoxy-bicyclo[4.2.0]octa-1,3,5-triene-7-ylmethyl)-methyl-amino]-propyl}-7,8-dimethoxy-1,3,4,5-tetrahydro-2H-3-benzazepine-2-one hydrobromide, **characterized by** that
a) 3-(2-bromo-4,5-dimethoxy-phenyl)-propionitrile of formula **(II)** 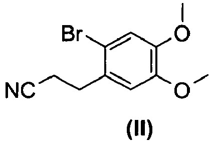 is cyclized with alkali-alkylamide of formula M-NR⁴R⁵ (R⁴ and R⁵ are hydrogen, C₁-C₄ straight or branched alkyl group, substituted alkyl group respectively or R⁴ and R⁵ are C₄-C₆ alkylidene group which can be unsaturated heterocycle ring. R⁴ and R⁵ together can also be a C₄-C₆ alkylidene group which can form a heterocycle ring with nitrogen and M is an alkali metal), and
b) the obtained 3,4-dimethoxy-bicyclo[4.2.0]octa-1,3,5-triene-7-carbonitrile compound of formula **(III)** is hydrolyzed, 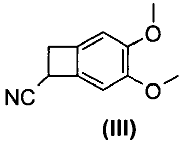
c) the obtained 3,4-dimethoxy-bicyclo [4.2.0]octa-1,3,5-triene-7-carboxylic acid compound of formula **(IV)** 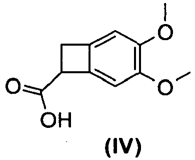 is resolved with chiral base of R¹R²R³N^{*} wherein R¹, R² and R³ are hydrogen, chiral or achiral C₁-C₄ alkyl, substituted chiral or achiral alkyl, chiral or achiral arylalkyl respectively, or wherein the R¹R²R³N chiral base can also be natural alkaloid or the mixture, the derivatives or the mixture of derivatives thereof,
d) in case the diastereomer salt (**Vb**) crystallizes, then the carboxylic acid of formula (**VIb**) is released from the obtained crystalline diastereomer salt of formula **(Vb):** 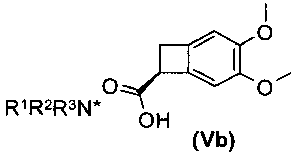
e) or in case the diastereomer salt of formula **(Va)** crystallizes, then the carboxylic acid enantiomer of formula (**VIb**) is obtained from the filtrate, 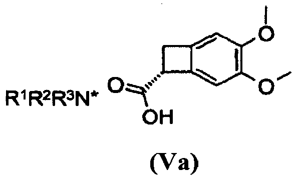
f) the corresponding acid chloride of (*S*)-3,4-dimethoxy-bicyclo[4.2.0]octa-1,3,5-triene-7-carboxylic acid compound of formula (**VIb**) is formed *in situ* and without isolation it is reacted with methylamine, 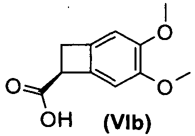
g) the obtained (*S*)-3,4-dimethoxy-bicyclo[4.2.0]octa-1,3,5-triene-7-carboxylic acid - methylamide of formula (**VII**) is reduced 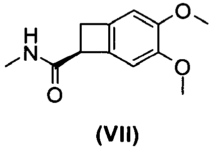
h) and ((*S*)-3,4-dimethoxy-bicyclo[4.2.0]octa-1,3,5-triene-7-yl-methyl)-methyl-amine hydrochloride of formula (**VIII**) is isolated, 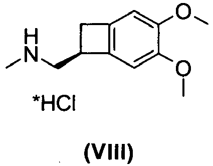 and it is reacted with 1-(7,8-dimethoxy-1,3-dihydro-2H-3-benzazepine-2-on-3-yl)-3-chloro-propane of formula (**IX**) 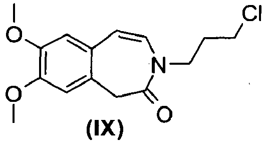
i) and the obtained 3-{3-[((S)-3,4-dimethoxy-bicyclo [4.2.0] octa-1,3,5-triene-7-ylmethyl)-methyl-amino]-propyl}-7,8-dimethoxy-1 ,3-dihydro-2H-3 -benzazepine-2-on of formula (**X**) 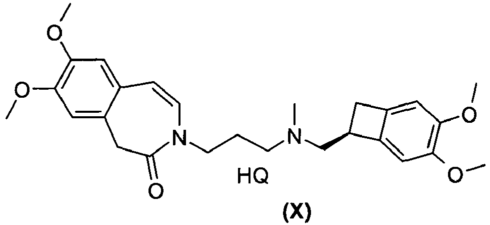 is isolated in the form of the nitrate or oxalate salt of general formula HQ with HQ=HNO₃ or (COOH)₂ and purified by recrystallization and from 3-{3-[((S)-3,4-dimethoxy-bicyclo[4.2.0] octa-1,3,5-triene-7-ylmethyl)-methyl-amino]-propyl}-7,8-dimethoxy-1,3-dihydro-2H-3-benzazepine-2-one salt of formula (**X**) the base is released with K₂CO₃ with or without isolation,
j) the base is subjected to catalytic hydrogenation with Pd-C catalyst in an ethanol or methanol solvent at the pressure of 1-15 bar at temperature of 25-100°C, then the Ivabradine hydrobromide salt of formula (**I**) is isolated, wherein the crystallization Ivabradine hydrobromide is carried out by cooling the methanol or ethanol solution.

2. Process according to Claim 1, **characterized by** that lithium diisopropyl amide is used as alkali-alkylamide reagent.

3. Process according to Claim 1, **characterized by** that 0.2 - 1.2 equivalent of R¹R²R³N chiral base is added to the racemic acid of formula (**IV**).

4. Process according Claim 1, **characterized by** that 0.4 - 0.6 equivalent of R¹R²R³N chiral base is used for the reaction.

5. Crystalline Ivabradine hydrobromide salt of formula (**I**) (HQ=HBr) **characterized by** at least one of the following characteristics:
• XRPD reflections measured at about: 5.0; 17.8; 18.5; 22.6; 23.8 and 25.0 °2θ
• IR absorption bands measured at about: 2930; 1664; 1522; 1463; 1219; and 1105 cm⁻¹
• Raman absorption bands measured at about: 2948; 2909; 1607; 1588; 1317 and 701 cm⁻¹.

6. 3-{3-[((*S*)-3,4-dimethoxy-bicyclo[4.2.0]octa-1,3,5-triene-7-ylmethyl)-methyl-amino]-propyl}-7,8-dimethoxy-1,3-dihydro-benzo[d]azepin-2-one oxalate of formula (**X**, HQ=(COOH)₂)

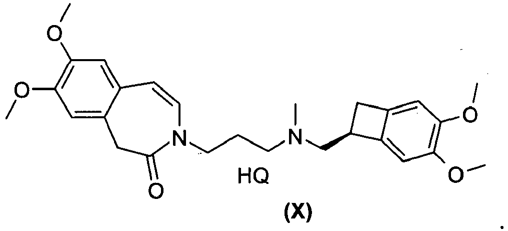

7. 3- {3-[((*S*)-3,4-dimethoxy-bicyclo[4.2.0]octa-1,3,5-triene-7-ylmethyl)-methyl-amino]-propyl}-7,8-dimethoxy-1,3-dihydro-benzo[d]azepin-2-one nitrate of formula (**X**, HQ=HNO₃)

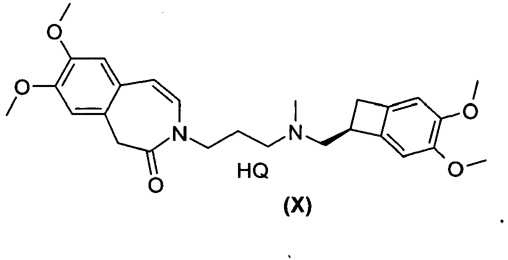

## Patentansprüche

1. Verfahren zur Synthese von Ivabradinhydrobromid der Formel (I), wobei HQ = HBr, 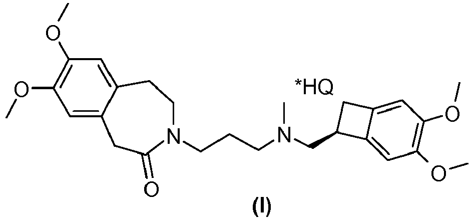 dessen chemischer Name ist: 3-{3-[((*S*)-3,4-Dimethoxybicyclo[4.2.0]octa-1,3,5-trien-7-ylmethyl)-methylamino]-propyl}-7,8-dimethoxy-1,3,4,5-tetrahydro-2H-3-benzazepin-2-onhydrobromid, **dadurch gekennzeichnet, dass**
a) 3-(2-Brom-4,5-dimethoxyphenyl)-propionitril der Formel (**II**) 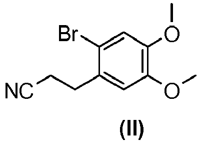 mit Alkali-Alkylamid der Formel M-NR⁴R⁵ zyklisiert wird
(R⁴ und R⁵ Wasserstoff, eine geradkettige oder verzweigte C₁-C₄-Alkylgruppe bzw. eine substituierte Alkylgruppe sind oder R⁴ und R⁵ eine C₄-C₆-Alkylidengruppe sind, die ein ungesättigter Heterozyklusring sein kann. R⁴ und R⁵ zusammen auch eine C₄-C₆-Alkylidengruppe sein können, die mit Stickstoff einen Heterozyklusring bilden kann, und M ein Alkalimetall ist) und
b) die erhaltene 3,4-Dimethoxybicyclo[4.2.0]octa-1,3,5-trien-7-carbonitril-Verbindung der Formel (**III**) hydrolysiert wird, 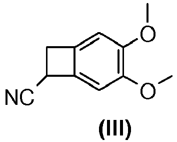
c) die gewonnene 3,4-Dimethoxybicyclo[4.2.0]octa-1,3,5-trien-7-carbonsäureverbindung der Formel (**IV**) 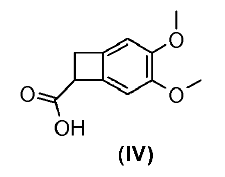 mit chiraler Base von R¹R²R³N^{*} aufgelöst wird, worin R¹, R² und R³ Wasserstoff, chirales oder achirales C₁-C₄-Alkyl, substituiertes chirales oder achirales Alkyl, chirales bzw. achirales Arylalkyl sind, oder worin die chirale R¹R²R³N-Base auch ein natürliches Alkaloid oder das Gemisch, die Derivate oder das Gemisch von Derivaten davon sein kann,
d) falls das Diastereomersalz (**Vb**) kristallisiert, dann wird die Karbonsäure der Formel **(VIb)** aus dem gewonnenen kristallinen Diastereomersalz der Formel (**Vb**) freigesetzt. 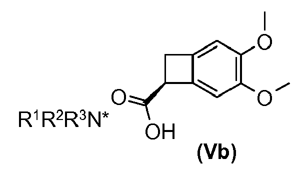
e) oder falls das Diastereomeresalz der Formel (**Va**) kristallisiert, dann wird das Carbonsäure-Enantiomer der Formel (**VIb**) aus dem Filtrat gewonnen, 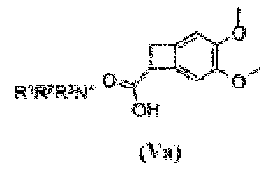
f) das entsprechende Säurechlorid der (*S*)-3,4-Dimethoxybicyclo[4.2.0]octa-1,3,5-trien-7-carbonsäureverbindung der Formel (**VIb**) *in situ* gebildet wird und ohne Isolierung mit Methylamin umgesetzt wird, 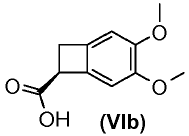
g) das gewonnene (*S*)-3,4-Dimethoxybicyclo[4.2.0]octa-1,3,5-trien-7-carbonsäuremethylamid der Formel (**VII**) reduziert wird 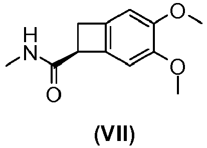
h) und ((*S*)-3,4-Dimethoxybicyclo[4.2.0]octa-1,3,5-trien-7-yl-methyl)methylaminhydrochlorid der Formel (**VIII**) isoliert wird, 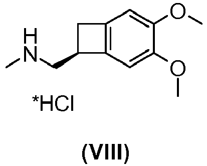 und es mit 1-(7,8-Dimethoxy-1,3-dihydro-2H-3-benzazepin-2-on-3-yl)-3-chlorpropan der Formel (**IX**) umgesetzt wird 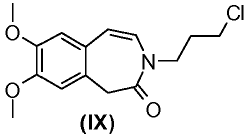
i) und das gewonnene 3-{3-[((S)-3,4-Dimethoxybicyclo[4.2.0]octa-1,3,5-trien-7-ylmethyl)-methyl-amino]-propyl}-7,8-dimethoxy-1,3-dihydro-2H-3-benzazepin-2-on der Formel (**X**) 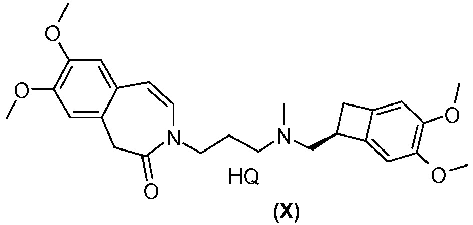 in Form des Nitrat- oder Oxalatsalzes der allgemeinen Formel HQ mit HQ = HNO₃ oder (COOH)₂ isoliert und durch Umkristallisation gereinigt wird und aus 3-{3-[((S)-3,4-Dimethoxybicyclo[4.2.0]octa-1,3,5-trien-7-ylmethyl)-methylamino]-propyl}-7,8-dimethoxy-1,3-dihydro-2H-3-benzazepin-2-on-Salz von Formel (**X**) die Base mit K₂CO₃ mit oder ohne Isolierung freigesetzt wird,
j) die Base einer katalytischen Hydrierung mit Pd-C-Katalysator in einem Ethanol- oder Methanollösungsmittel bei einem Druck von 1-15 bar bei einer Temperatur von 25-100 °C unterzogen wird, dann das Ivabradinhydrobromidsalz der Formel (I) isoliert wird, wobei die Kristallisation von Ivabradinhydrobromid durch Abkühlen der Methanol- oder Ethanollösung durchgeführt wird.

2. Verfahren nach Anspruch 1, **dadurch gekennzeichnet, dass** Lithiumdiisopropylamid als Alkali-Alkylamid-Reagens verwendet wird.

3. Verfahren nach Anspruch 1, **dadurch gekennzeichnet, dass** 0,2-1,2 Äquivalente der chiralen R¹R²R³N-Base zu der racemischen Säure der Formel (**IV**) hinzugefügt werden.

4. Verfahren nach Anspruch 1, **dadurch gekennzeichnet, dass** 0,4-0,6 Äquivalente der chiralen R¹R²R³N-Base für die Reaktion verwendet werden.

5. Kristallines Ivabradinhydrobromidsalz der Formel (**I**) (HQ = HBr), **gekennzeichnet durch** mindestens eine der folgenden Eigenschaften:
• XRPD-Reflexionen gemessen bei etwa: 5,0; 17,8; 18,5; 22,6; 23,8 und 25,0 °2θ
• IR-Absorptionsbanden gemessen bei etwa: 2930; 1664; 1522; 1463; 1219; und 1105 cm⁻¹
• Raman-Absorptionsbanden bei etwa: 2948; 2909; 1607; 1588; 1317 und 701 cm⁻¹.

6. 3-{3-[((*S*)-3,4-Dimethoxybicyclo[4.2.0]octa-1,3,5-trien-7-ylmethyl)-methylamino]-propyl}-7,8-dimethoxy-1,3-dihydrobenzo[d]azepin-2-onoxalat der Formel (**X**, HQ=(COOH)₂).

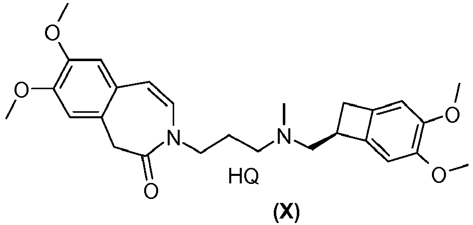

7. 3-{3-[((*S*)-3,4-Dimethoxybicyclo[4.2.0]octa-1,3,5-trien-7-ylmethyl)-methylamino]-propyl}-7,8-dimethoxy-1,3-dihydrobenzo[d]azepin-2-onnitrat der Formel (**X**, HQ.=HNO₃).

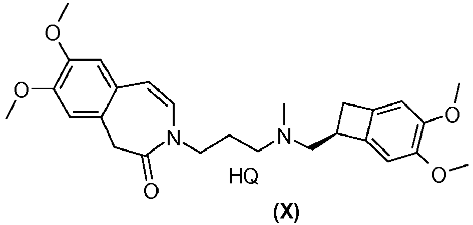

## Revendications

1. Procédé pour la synthèse de bromhydrate d'ivabradine répondant à la formule (I), avec HQ=HBr, 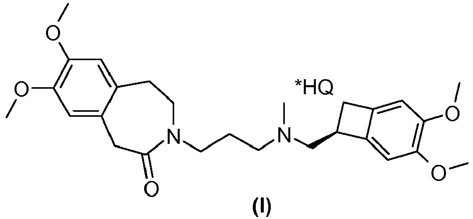 dont le nom chimique est : le bromhydrate de 3-{3-[((*S*)-3,4-diméthoxy-bicyclo[4.2.0]octa-1,3,5-triène-7-ylméthyl)-méthylamino]-propyl}-7,8-diméthoxy-1,3,4,5-tétrahydro-2H-3-benzazépine-2-one, **caractérisé en ce que**
a) le 3-(2-bromo-4,5-diméthoxy-phényl)-propionitrile répondant à la formule (II) 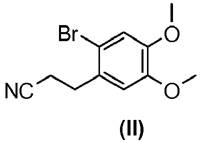 est cyclisé avec un alkylamide alcalin répondant à la formule M-NR⁴R⁵ (R⁴ et R⁵ sont l'hydrogène, un groupe alkyle linéaire ou ramifié en C₁-C₄, un groupe alkyle substitué respectivement ou R⁴ et R⁵ sont un groupe alkylidène en C₄-C₆ qui peut être un noyau hétérocyclique insaturé. R⁴ et R⁵ ensemble peuvent également être un groupe alkylidène en C₄-C₆ qui peut former un noyau hétérocyclique avec de l'azote et M est un métal alcalin), et
b) le composé 3,4-diméthoxy-bicyclo[4.2.0]octa-1,3,5-triène-7-carbonitrile obtenu répondant à la formule (III) est hydrolysé, 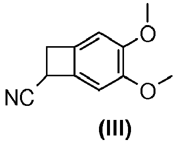
c) le composé acide 3,4-diméthoxy-bicyclo[4.2.0]octa-1,3,5-triène-7-carboxylique obtenu répondant à la formule (IV) 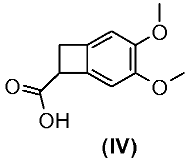 est résolu avec une base chirale de R¹R²R³N^{*} dans laquelle R¹, R² et R³ sont l'hydrogène, un alkyle chiral ou achiral en C₁-C₄, un alkyle chiral ou achiral substitué, un arylakyle chiral ou achiral respectivement, ou dans laquelle la base chirale R¹R²R³N peut également être un alcaloïde naturel ou le mélange, les dérivés ou le mélange des dérivés de celui-ci,
d) dans le cas où le sel diastéréomère (Vb) cristallise, l'acide carboxylique répondant à la formule (VIb) est libéré du sel diastéréomètre cristallin répondant obtenu à la formule (Vb) 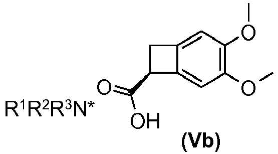
e) ou dans le cas où le sel diastéréomère répondant à la formule (Va) cristallise, l'énantiomère d'acide carboxylique répondant à la formule (VIb) est obtenu du filtrat, 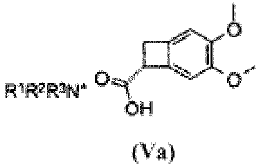
f) le chlorure d'acide correspondant du composé acide (*S*)-3,4-diméthoxy-bicyclo[4.2.0]octa-1,3,5-triène-7-carboxylique répondant à la formule (VIb) est formé *in situ* et sans être isolé il est mis à réagir avec de la méthylamine, 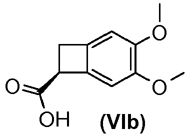
g) le méthylamide d'acide (*S*)-3,4-diméthoxy-bicyclo[4.2.0]octa-1,3,5-triène-7-carboxylique répondant à la formule (VII) obtenu est réduit 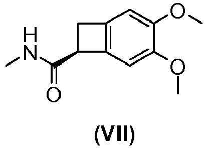
h) et le chlorhydrate de ((*S*)-3,4-diméthoxy-bicyclo[4.2.0]octa-1,3,5-triène-7-yl-méthyl)-méthylamine répondant à la formule (VIII) est isolé, 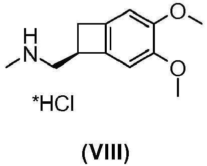 et est mis à réagir avec le 1-(7,8-diméthoxy-1,3-dihydro-2H-3-benzazépine-2-on-3-yl)-3-chloro-propane répondant à la formule (IX) 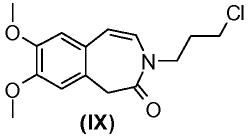
i) et la 3-{3-[((S)-3,4-diméthoxy-bicyclo [4.2.0]octa-1,3,5-triène-7-ylméthyl)-méthylamino]-propyl}-7,8-diméthoxy-1,3-dihydro-2H-3-benzazépine-2-one obtenue répondant à la formule (X) 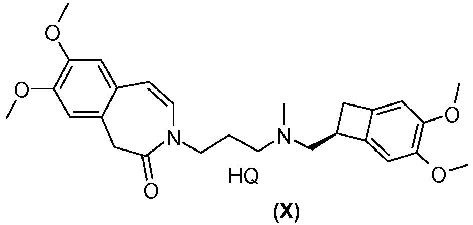 est isolée sous la forme du nitrate ou du sel d'oxalate répondant à la formule générale HQ avec HO=HNO₃ ou (COOH)₂ et purifiée par recristallisation et à partir de sel de 3-{3-[((S)-3,4-diméthoxy-bicyclo[4.2.0]octa-1,3,5-triène-7-ylméthyl)-méthylamino]-propyl}-7,8-diméthoxy-1,3-dihydro-2H-3-benzazépine-2-one répondant à la formule (X) la base est libérée avec K₂CO₃ avec ou sans isolement,
j) la base est soumise à une hydrogénation catalytique avec un catalyseur au Pd-C dans un solvant à l'éthanol ou au méthanol à la pression de 1 à 15 bars à une température de 25 à 100 °C, puis le sel de bromhydrate d'ivabradine répondant à la formule (I) est isolé, la cristallisation du bromhydrate d'ivabradine étant réalisée en refroidissant la solution de méthanol ou d'éthanol.

2. Procédé selon la revendication 1, **caractérisé en ce qu'**un amide diisopropylique de lithium est utilisé comme réactif alkylamide alcalin.

3. Procédé selon la revendication 1, **caractérisé en ce que** 0,2 - 1,2 équivalent de base chirale R¹R²R³N est ajouté à l'acide racémique répondant à la formule (IV).

4. Procédé selon la revendication 1, **caractérisé en ce que** 0,4 - 0,6 équivalent de base chirale R¹R²R³N est utilisé pour la réaction.

5. Sel de bromhydrate d'ivabradine cristallin répondant à la formule (I) (HQ=HBr) **caractérisé par** au moins une des caractéristiques suivantes :
• des réflexions XRPD mesurées à environ: 5,0 ; 17,8 ; 18,5 ; 22,6 ; 23,8 et 25,0 °2θ
• des bandes d'absorption IR mesurées à environ : 2 930 ; 1664; 1 522 ; 1463 ; 1 219 ; et 1105 cm⁻¹
• des bandes d'absorption Raman mesurées à environ : 2 948 ; 2 909 ; 1 607 ; 1588 ; 1 317 et 701 cm⁻¹.

6. Oxalate de 3-{3-[((*S*)-3,4-diméthoxy-bicyclo[4.2.0]octa-1,3,5-triène-7-ylméthyl)-méthyl-amino]-propyl}-7,8-diméthoxy-1,3-dihydro-benzo[d]azépin-2-one répondant à la formule (X, HQ=(COOH)₂).

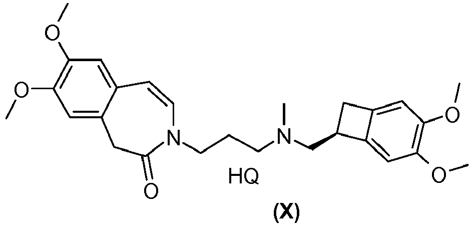

7. Nitrate de 3-{3-[((S)-3,4-diméthoxy-bicyclo [4.2.0]octa-1,3,5-triène-7-ylméthyl)-méthylamino]-propyl}-7,8-diméthoxy-1,3-dihydro-benzo[d]azépin-2-one répondant à la formule (X, HQ=HNO₃).
